(19) Europäisches Patentamt
European Patent Office
Office européen des brevets

(11) EP 4 782 006 A1

(12) EUROPEAN PATENT APPLICATION
published in accordance with Art. 153(4) EPC

(43) Date of publication:
**29.07.2026 Bulletin 2026/31**

(21) Application number: **24867510.0**

(22) Date of filing: **19.09.2024**

(51) International Patent Classification (IPC):
***A61K 45/06*** (2006.01) ***A61K 31/4418*** (2006.01)
***A61K 31/4545*** (2006.01) ***A61P 31/06*** (2006.01)

(52) Cooperative Patent Classification (CPC):
**A61K 31/4418; A61K 31/4545; A61K 45/06; A61P 31/06**

(86) International application number:
**PCT/CN2024/119801**

(87) International publication number:
**WO 2025/061104 (27.03.2025 Gazette 2025/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.09.2023 CN 202311228851**

(71) Applicants:
- **Guangzhou Joyo Pharmatech Co., Ltd.
Guangzhou, Guangdong 510663 (CN)**
- **BEIJING CHEST HOSPITAL AFFILIATED OF CAPITAL MEDICAL UNIVERSITY
Beijing 101100 (CN)**
- **Shanghai Jia Tan Pharmatech Co. Ltd.
Shanghai 201203 (CN)**

(72) Inventors:
- **LU, Yu
Beijing 101100 (CN)**
- **FU, Lei
Beijing 101100 (CN)**
- **YAO, Rong
Beijing 101100 (CN)**
- **ZHANG, Weiyan
Beijing 101100 (CN)**
- **WANG, Bin
Beijing 101100 (CN)**
- **CHEN, Xi
Beijing 101100 (CN)**
- **WANG, Ning
Beijing 101100 (CN)**
- **DING, Yangming
Beijing 101100 (CN)**
- **ZHANG, Lei
Beijing 101100 (CN)**
- **LI, Zongrui
Guangzhou, Guangdong 510663 (CN)**
- **CHEN, Xiaoning
Guangzhou, Guangdong 510663 (CN)**
- **LI, Lei
Guangzhou, Guangdong 510663 (CN)**
- **LI, Yongguo
Guangzhou, Guangdong 510663 (CN)**

(74) Representative: **Michalski Hüttermann & Partner mbB
Kaistraße 16A
40221 Düsseldorf (DE)**

# (54) PHARMACEUTICAL COMBINATION AND USE

(57) Disclosed in the present invention are a pharmaceutical combination containing a substance X, a substance Y and an optional substance Z, and the use thereof in the preparation of drugs for treating diseases caused by Mycobacterium tuberculosis. The substance X is a compound I or a pharmaceutically acceptable salt thereof, and the substance Y and the substance Z are selected from pyrazinamide, linezolid, moxifloxacin, pretomanid or delamanid.

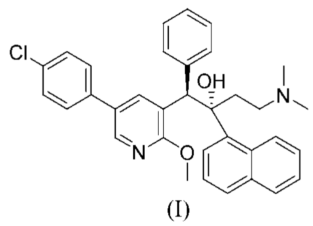

(I)

EP 4 782 006 A1

## Description

The present application claims priority to Chinese Patent Application No. 2023112288517, filed on September 21, 2023. The aforementioned Chinese patent application is incorporated herein by reference in its entirety.

## TECHNICAL FIELD

The present disclosure relates to a pharmaceutical combination and a use thereof. Specifically, the present disclosure relates to a pharmaceutical combination for treating a disease caused by Mycobacterium tuberculosis.

## BACKGROUND

Tuberculosis (TB) is a chronic infectious disease caused by *Mycobacterium tuberculosis* (MTB) infection and remains the single infectious disease responsible for the highest number of deaths to date. A major challenge in achieving the global goal of ending the tuberculosis epidemic by 2035 is the rising incidence of drug-resistant and multidrug-resistant tuberculosis. Currently, tuberculosis treatment has a long cycle and the cure rate for multidrug-resistant tuberculosis is low. There is an urgent need for new drugs with novel mechanisms of action for the treatment of tuberculosis, especially drug-resistant tuberculosis, as well as an urgent need to develop new chemotherapy regimens that shorten the treatment course, simplify treatment, and improve efficacy.

Due to the presence of different bacterial populations in tuberculosis lesions and the rapid development of resistance by MTB to a single drug, drug combination is a key weapon in the treatment of tuberculosis; that is, tuberculosis treatment must be carried out in the form of combination medication. In addition, the clearance of dormant MTB persisting in the host is a key factor determining the length of the tuberculosis chemotherapy course. Therefore, the development of new and effective combination treatment regimens is crucial for shortening the treatment course of tuberculosis. The development of novel anti-tuberculosis combination regimens can improve efficacy and shorten treatment time, which will improve patient compliance, reduce side effects, lower costs, and increase the cure rate. Therefore, there is a need in the art for novel regimens that are more effective than current first-line regimens and are thus unaffected by resistance to existing drugs.

Compound I (WX-081) possesses good anti-tuberculosis activity both *in vivo* and *in vitro.* Currently, compound I has entered a Phase III pivotal clinical study (CTR20221162). To date, there have been no reports at home or abroad regarding the combined application of compound I with other anti-tuberculosis drugs.

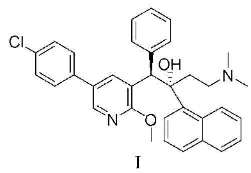

I

## SUMMARY

The present disclosure provides a pharmaceutical combination, which can prevent and/or treat a disease caused by *Mycobacterium tuberculosis,* and a use thereof.

In a first aspect, the present disclosure provides a pharmaceutical combination comprising:

i) substance X, wherein the substance X is compound I or a pharmaceutically acceptable salt thereof;
ii) substance Y, wherein the substance Y is a second therapeutic agent or a pharmaceutically acceptable salt thereof, and the second therapeutic agent is pyrazinamide, linezolid, moxifloxacin, pretomanid, or delamanid;
the structure of compound I is shown below:

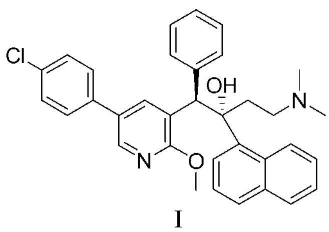

I .

In some embodiments, the substance X is a pharmaceutically acceptable salt of compound I; preferably, the substance X is a hydrochloride, sulfate, citrate, maleate, or fumarate salt of compound I; more preferably, the substance X is a fumarate salt of compound I.

In some embodiments, the substance X is compound I.

In some embodiments, the second therapeutic agent is selected from pyrazinamide, moxifloxacin, delamanid, and pretomanid.

In some embodiments, the second therapeutic agent is delamanid or pyrazinamide.

In some embodiments, the pharmaceutical combination consists of the substance X and the substance Y.

In some embodiments, the substance X and the substance Y are administered simultaneously or separately.

In some embodiments, the substance X is administered orally.

In some embodiments, the substance Y is administered orally or by injection; preferably, the substance Y is administered orally.

In a second aspect, the present disclosure provides a pharmaceutical composition A comprising substance X, substance Y, and a pharmaceutically acceptable carrier or medium, wherein the substance X is compound I or a pharmaceutically acceptable salt thereof; the substance Y is a second therapeutic agent or a pharmaceutically acceptable salt thereof, and the second therapeutic agent is pyrazinamide, linezolid, moxifloxacin, pretomanid, or delamanid;
the structure of compound I is shown below:

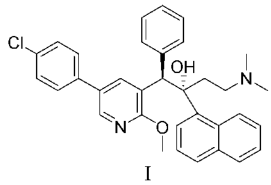

I

In some embodiments, the substance X is compound I.

In some embodiments, the substance X is a pharmaceutically acceptable salt of compound I; preferably, the substance X is a hydrochloride, sulfate, citrate, maleate, or fumarate salt of compound I; more preferably, the substance X is a fumarate salt of compound I.

In some embodiments, the substance Y is a second therapeutic agent or a pharmaceutically acceptable salt thereof, and the second therapeutic agent is pyrazinamide, linezolid, moxifloxacin, pretomanid, or delamanid.

In some embodiments, the second therapeutic agent is pyrazinamide or delamanid.

In some embodiments, the pharmaceutical composition A is presented in an oral dosage form.

In some embodiments, the pharmaceutical composition A consists of the substance X, the substance Y, and a pharmaceutically acceptable carrier or medium.

In a third aspect, the present disclosure provides a pharmaceutical composition B comprising a first pharmaceutical composition and a second pharmaceutical composition;

the first pharmaceutical composition comprises substance X and a pharmaceutically acceptable carrier or medium, wherein the substance X is compound I or a pharmaceutically acceptable salt thereof;
the second pharmaceutical composition comprises substance Y and a pharmaceutically acceptable carrier or medium, wherein the substance Y is a second therapeutic agent or a pharmaceutically acceptable salt thereof, and the second therapeutic agent is pyrazinamide, linezolid, moxifloxacin, pretomanid, or delamanid;
the structure of compound I is shown below:

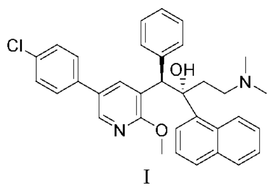

I .

In some embodiments, the substance X is compound I.

In some embodiments, the substance X is a pharmaceutically acceptable salt of compound I; preferably, the substance X is a hydrochloride, sulfate, citrate, maleate, or fumarate salt of compound I; more preferably, the substance X is a fumarate salt of compound I.

In some embodiments, the substance Y is a second therapeutic agent or a pharmaceutically acceptable salt thereof, and the second therapeutic agent is pyrazinamide, linezolid, moxifloxacin, pretomanid, or delamanid.

In some embodiments, the second therapeutic agent is pyrazinamide or delamanid.
In some embodiments, the first pharmaceutical composition is presented in an oral dosage form.
In some embodiments, the second pharmaceutical composition is presented in an oral dosage form.
In some embodiments, the pharmaceutical composition B consists of the first pharmaceutical composition and the second pharmaceutical composition.
In a fourth aspect, the present disclosure provides a pharmaceutical combination comprising:

i) substance X, wherein the substance X is compound I or a pharmaceutically acceptable salt thereof;
ii) substance Y, wherein the substance Y is a second therapeutic agent or a pharmaceutically acceptable salt thereof, and the second therapeutic agent is pretomanid or delamanid; and
iii) substance Z, wherein the substance Z is linezolid or a pharmaceutically acceptable salt thereof;
the structure of compound I is shown below:

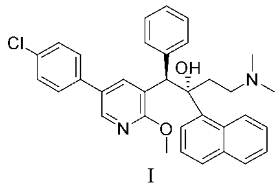

I .

In some embodiments, the substance X is compound I.
In some embodiments, the substance X is a pharmaceutically acceptable salt of compound I; preferably, the substance X is a hydrochloride, sulfate, citrate, maleate, or fumarate salt of compound I; more preferably, the substance X is a fumarate salt of compound I.
In some embodiments, the second therapeutic agent is pretomanid.
In some embodiments, the pharmaceutical combination consists of the substance X, the substance Y, and the substance Z.
In some embodiments, the substance X, the substance Y, and the substance Z are administered simultaneously or separately.
In some embodiments, the substance X is administered orally.
In some embodiments, the substance Y is administered orally.
In some embodiments, the substance Z is administered orally or by injection; preferably, the substance Z is administered orally.
In some embodiments, the pharmaceutical combination comprises 100 mg to 1000 mg of a compound of formula (I) or a pharmaceutically acceptable salt thereof.
In some embodiments, the pharmaceutical combination comprises 100 mg to 800 mg of the compound of formula (I) or a pharmaceutically acceptable salt thereof.
In some embodiments, the pharmaceutical combination comprises 150 mg to 800 mg of the compound of formula (I) or a pharmaceutically acceptable salt thereof.
In some embodiments, the pharmaceutical combination comprises 150 mg to 500 mg of the compound of formula (I) or a pharmaceutically acceptable salt thereof.
In some embodiments, the pharmaceutical combination comprises 150 mg to 450 mg of a compound of formula (I) or a pharmaceutically acceptable salt thereof.
In some embodiments, the pharmaceutical combination comprises 150 mg, 200 mg, 250 mg, 300 mg, 350 mg, 400 mg, or 450 mg of the compound of formula (I) or a pharmaceutically acceptable salt thereof.
In some embodiments, the pharmaceutical combination comprises 450 mg of the compound of formula (I) or a pharmaceutically acceptable salt thereof.
In some embodiments, the pharmaceutical combination comprises 300 mg of the compound of formula (I) or a pharmaceutically acceptable salt thereof.
In some embodiments, the pharmaceutical combination comprises 150 mg of the compound of formula (I) or a pharmaceutically acceptable salt thereof.
In some embodiments, the second therapeutic agent is pretomanid, and the pharmaceutical combination comprises 25 mg to 1000 mg of pretomanid or a pharmaceutically acceptable salt thereof.
In some embodiments, the second therapeutic agent is pretomanid, and the pharmaceutical combination comprises 50 mg to 1000 mg of pretomanid or a pharmaceutically acceptable salt thereof.
In some embodiments, the second therapeutic agent is pretomanid, and the pharmaceutical combination comprises 50 mg to 800 mg of pretomanid or a pharmaceutically acceptable salt thereof.
In some embodiments, the second therapeutic agent is pretomanid, and the pharmaceutical combination

comprises 50 mg to 600 mg of pretomanid or a pharmaceutically acceptable salt thereof.

In some embodiments, the second therapeutic agent is pretomanid, and the pharmaceutical combination comprises 100 mg to 800 mg of pretomanid or a pharmaceutically acceptable salt thereof.

In some embodiments, the second therapeutic agent is pretomanid, and the pharmaceutical combination comprises 100 mg to 600 mg of pretomanid or a pharmaceutically acceptable salt thereof.

In some embodiments, the second therapeutic agent is pretomanid, and the pharmaceutical combination comprises 150 mg to 600 mg of pretomanid or a pharmaceutically acceptable salt thereof.

In some embodiments, the second therapeutic agent is pretomanid, and the pharmaceutical combination comprises 200 mg to 600 mg of pretomanid or a pharmaceutically acceptable salt thereof.

In some embodiments, the second therapeutic agent is pretomanid, and the pharmaceutical combination comprises 100 mg, 200 mg, 300 mg, 400 mg, 500 mg, or 600 mg of pretomanid or a pharmaceutically acceptable salt thereof.

In some embodiments, the pharmaceutical combination comprises 50 mg to 2000 mg of linezolid or a pharmaceutically acceptable salt thereof.

In some embodiments, the pharmaceutical combination comprises 10 mg to 1200 mg of linezolid or a pharmaceutically acceptable salt thereof.

In some embodiments, the pharmaceutical combination comprises 50 mg to 1200 mg of linezolid or a pharmaceutically acceptable salt thereof.

In some embodiments, the pharmaceutical combination comprises 100 mg to 1200 mg of linezolid or a pharmaceutically acceptable salt thereof.

In some embodiments, the pharmaceutical combination comprises 200 mg to 1200 mg of linezolid or a pharmaceutically acceptable salt thereof.

In some embodiments, the pharmaceutical combination comprises 50 mg to 1000 mg of linezolid or a pharmaceutically acceptable salt thereof.

In some embodiments, the pharmaceutical combination comprises 100 mg to 1000 mg of linezolid or a pharmaceutically acceptable salt thereof.

In some embodiments, the pharmaceutical combination comprises 150 mg to 1000 mg of linezolid or a pharmaceutically acceptable salt thereof.

In some embodiments, the pharmaceutical combination comprises 150 mg to 800 mg of linezolid or a pharmaceutically acceptable salt thereof.

In some embodiments, the pharmaceutical combination comprises 150 mg to 600 mg of linezolid or a pharmaceutically acceptable salt thereof.

In some embodiments, the pharmaceutical combination comprises 200 mg to 600 mg of linezolid or a pharmaceutically acceptable salt thereof.

In some embodiments, the pharmaceutical combination comprises 100 mg, 200 mg, 300 mg, 400 mg, 500 mg, 600 mg, 700 mg, 800 mg, 900 mg, 1000 mg, 1200 mg, or 1600 mg of linezolid or a pharmaceutically acceptable salt thereof.

In some embodiments, the pharmaceutical combination comprises 150 mg to 450 mg of the compound of formula (I) or a pharmaceutically acceptable salt thereof, 200 mg to 600 mg of pretomanid or a pharmaceutically acceptable salt thereof, and 200 mg to 1200 mg (preferably 200 mg to 600 mg) of linezolid or a pharmaceutically acceptable salt thereof.

In some embodiments, the pharmaceutical combination comprises: (1) 150 mg, 200 mg, 250 mg, 300 mg, 350 mg, 400 mg, or 450 mg of the compound of formula (I) or a pharmaceutically acceptable salt thereof; (2) 100 mg, 200 mg, 300 mg, 400 mg, 500 mg, or 600 mg of pretomanid or a pharmaceutically acceptable salt thereof; and (3) 200 mg, 300 mg, 400 mg, 500 mg, 600 mg, or 1200 mg of linezolid or a pharmaceutically acceptable salt thereof.

In a fifth aspect, the present disclosure provides a pharmaceutical composition C comprising substance X, substance Y, substance Z, and a pharmaceutically acceptable carrier or medium, wherein the substance X is compound I or a pharmaceutically acceptable salt thereof;

the substance Y is a second therapeutic agent or a pharmaceutically acceptable salt thereof, and the second therapeutic agent is pretomanid or delamanid;

the substance Z is linezolid or a pharmaceutically acceptable salt thereof;

the structure of compound I is shown below:

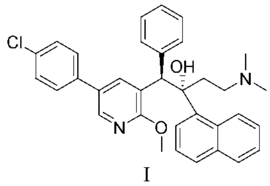

I .

In some embodiments, the substance X is compound I.

In some embodiments, the substance X is a pharmaceutically acceptable salt of compound I; preferably, the substance X is a hydrochloride, sulfate, citrate, maleate, or fumarate salt of compound I; more preferably, the substance X is a fumarate salt of compound I.

In some embodiments, the second therapeutic agent is pretomanid.

In some embodiments, the pharmaceutical composition C is presented in an oral dosage form.

In some embodiments, the pharmaceutical composition C consists of the substance X, the substance Y, the substance Z, and a pharmaceutically acceptable carrier or medium.

In some embodiments, the pharmaceutical composition C comprises 100 mg to 1000 mg of the compound of formula (I) or a pharmaceutically acceptable salt thereof.

In some embodiments, the pharmaceutical composition C comprises 100 mg to 800 mg of the compound of formula (I) or a pharmaceutically acceptable salt thereof.

In some embodiments, the pharmaceutical composition C comprises 150 mg to 800 mg of the compound of formula (I) or a pharmaceutically acceptable salt thereof.

In some embodiments, the pharmaceutical composition C comprises 150 mg to 500 mg of the compound of formula (I) or a pharmaceutically acceptable salt thereof.

In some embodiments, the pharmaceutical composition C comprises 150 mg to 450 mg of the compound of formula (I) or a pharmaceutically acceptable salt thereof.

In some embodiments, the pharmaceutical composition C comprises 150 mg, 200 mg, 250 mg, 300 mg, 350 mg, 400 mg, or 450 mg of the compound of formula (I) or a pharmaceutically acceptable salt thereof.

In some embodiments, the pharmaceutical composition C comprises 450 mg of the compound of formula (I) or a pharmaceutically acceptable salt thereof.

In some embodiments, the pharmaceutical composition C comprises 300 mg of the compound of formula (I) or a pharmaceutically acceptable salt thereof.

In some embodiments, the pharmaceutical composition C comprises 150 mg of the compound of formula (I) or a pharmaceutically acceptable salt thereof.

In some embodiments, the second therapeutic agent is pretomanid, and the pharmaceutical composition C comprises 25 mg to 1000 mg of pretomanid or a pharmaceutically acceptable salt thereof.

In some embodiments, the second therapeutic agent is pretomanid, and the pharmaceutical composition C comprises 50 mg to 1000 mg of pretomanid or a pharmaceutically acceptable salt thereof.

In some embodiments, the second therapeutic agent is pretomanid, and the pharmaceutical composition C comprises 50 mg to 800 mg of pretomanid or a pharmaceutically acceptable salt thereof.

In some embodiments, the second therapeutic agent is pretomanid, and the pharmaceutical composition C comprises 50 mg to 600 mg of pretomanid or a pharmaceutically acceptable salt thereof.

In some embodiments, the second therapeutic agent is pretomanid, and the pharmaceutical composition C comprises 100 mg to 800 mg of pretomanid or a pharmaceutically acceptable salt thereof.

In some embodiments, the second therapeutic agent is pretomanid, and the pharmaceutical composition C comprises 100 mg to 600 mg of pretomanid or a pharmaceutically acceptable salt thereof.

In some embodiments, the second therapeutic agent is pretomanid, and the pharmaceutical composition C comprises 150 mg to 600 mg of pretomanid or a pharmaceutically acceptable salt thereof.

In some embodiments, the second therapeutic agent is pretomanid, and the pharmaceutical composition C comprises 200 mg to 600 mg of pretomanid or a pharmaceutically acceptable salt thereof.

In some embodiments, the second therapeutic agent is pretomanid, and the pharmaceutical composition C comprises 100 mg, 200 mg, 300 mg, 400 mg, 500 mg, or 600 mg of pretomanid or a pharmaceutically acceptable salt thereof.

In some embodiments, the pharmaceutical composition C comprises 50 mg to 2000 mg of linezolid or a pharmaceutically acceptable salt thereof.

In some embodiments, the pharmaceutical composition C comprises 10 mg to 1200 mg of linezolid or a pharmaceutically acceptable salt thereof.

In some embodiments, the pharmaceutical composition C comprises 50 mg to 1200 mg of linezolid or a pharmaceutically acceptable salt thereof.

In some embodiments, the pharmaceutical composition C comprises 100 mg to 1200 mg of linezolid or a pharmaceutically acceptable salt thereof.

In some embodiments, the pharmaceutical composition C comprises 200 mg to 1200 mg of linezolid or a pharmaceutically acceptable salt thereof.

In some embodiments, the pharmaceutical composition C comprises 10 mg to 1000 mg of linezolid or a pharmaceutically acceptable salt thereof.

In some embodiments, the pharmaceutical composition C comprises 50 mg to 1000 mg of linezolid or a pharmaceutically acceptable salt thereof.

In some embodiments, the pharmaceutical composition C comprises 100 mg to 1000 mg of linezolid or a pharmaceutically acceptable salt thereof.

In some embodiments, the pharmaceutical composition C comprises 150 mg to 1000 mg of linezolid or a pharmaceutically acceptable salt thereof.

In some embodiments, the pharmaceutical composition C comprises 150 mg to 800 mg of linezolid or a pharmaceutically acceptable salt thereof.

In some embodiments, the pharmaceutical composition C comprises 150 mg to 600 mg of linezolid or a pharmaceutically acceptable salt thereof.

In some embodiments, the pharmaceutical composition C comprises 200 mg to 600 mg of linezolid or a pharmaceutically acceptable salt thereof.

In some embodiments, the pharmaceutical composition C comprises 100 mg, 200 mg, 300 mg, 400 mg, 500 mg, 600 mg, 700 mg, 800 mg, 900 mg, 1000 mg, 1200 mg, or 1600 mg of linezolid or a pharmaceutically acceptable salt thereof.

In some embodiments, the pharmaceutical composition C comprises 150 mg to 450 mg of the compound of formula (I) or a pharmaceutically acceptable salt thereof, 200 mg to 600 mg of pretomanid or a pharmaceutically acceptable salt thereof, and 200 mg to 1200 mg (preferably 200 mg to 600 mg) of linezolid or a pharmaceutically acceptable salt thereof.

In some embodiments, the pharmaceutical composition C comprises: (1) 150 mg, 200 mg, 250 mg, 300 mg, 350 mg, 400 mg, or 450 mg of the compound of formula (I) or a pharmaceutically acceptable salt thereof; (2) 100 mg, 200 mg, 300 mg, 400 mg, 500 mg, or 600 mg of pretomanid or a pharmaceutically acceptable salt thereof; and (3) 200 mg, 300 mg, 400 mg, 500 mg, 600 mg, or 1200 mg of linezolid or a pharmaceutically acceptable salt thereof.

In a sixth aspect, the present disclosure provides a pharmaceutical composition D comprising a first pharmaceutical composition, a second pharmaceutical composition, and a third pharmaceutical composition;

the first pharmaceutical composition comprises substance X and a pharmaceutically acceptable carrier or medium, wherein the substance X is compound I or a pharmaceutically acceptable salt thereof;

the second pharmaceutical composition comprises substance Y and a pharmaceutically acceptable carrier or medium, wherein the substance Y is a second therapeutic agent or a pharmaceutically acceptable salt thereof, and the second therapeutic agent is pretomanid or delamanid;

the third pharmaceutical composition comprises substance Z and a pharmaceutically acceptable carrier or medium, wherein the substance Z is a third therapeutic agent or a pharmaceutically acceptable salt thereof, and the third therapeutic agent is linezolid;

the structure of compound I is shown below:

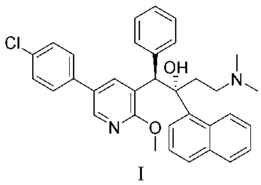

I .

In some embodiments, the substance X is compound I.

In some embodiments, the substance X is a pharmaceutically acceptable salt of compound I; preferably, the substance X is a hydrochloride, sulfate, citrate, maleate, or fumarate salt of compound I; more preferably, the substance X is a fumarate salt of compound I.

In some embodiments, the second therapeutic agent is pretomanid.

In some embodiments, the first pharmaceutical composition is presented in an oral dosage form.

In some embodiments, the second pharmaceutical composition is presented in an oral dosage form.

In some embodiments, the third pharmaceutical composition is presented in an oral dosage form.

In some embodiments, the pharmaceutical composition D consists of the first pharmaceutical composition, the second pharmaceutical composition, and the third pharmaceutical composition.

In some embodiments, the first pharmaceutical composition comprises 100 mg to 1000 mg of the compound of formula (I) or a pharmaceutically acceptable salt thereof.

In some embodiments, the first pharmaceutical composition comprises 100 mg to 800 mg of the compound of formula (I) or a pharmaceutically acceptable salt thereof.

In some embodiments, the first pharmaceutical composition comprises 150 mg to 800 mg of the compound of formula (I) or a pharmaceutically acceptable salt thereof.

In some embodiments, the first pharmaceutical composition comprises 150 mg to 500 mg of the compound of formula (I) or a pharmaceutically acceptable salt thereof.

In some embodiments, the first pharmaceutical composition comprises 150 mg to 450 mg of the compound of formula (I) or a pharmaceutically acceptable salt thereof.

In some embodiments, the first pharmaceutical composition comprises 150 mg, 200 mg, 250 mg, 300 mg, 350 mg, 400 mg, or 450 mg of the compound of formula (I) or a pharmaceutically acceptable salt thereof.

In some embodiments, the first pharmaceutical composition comprises 450 mg of the compound of formula (I) or a pharmaceutically acceptable salt thereof.

In some embodiments, the first pharmaceutical composition comprises 300 mg of the compound of formula (I) or a pharmaceutically acceptable salt thereof.

In some embodiments, the first pharmaceutical composition comprises 150 mg of the compound of formula (I) or a pharmaceutically acceptable salt thereof.

In some embodiments, the second therapeutic agent is pretomanid, and the second pharmaceutical composition comprises 25 mg to 1000 mg of pretomanid or a pharmaceutically acceptable salt thereof.

In some embodiments, the second therapeutic agent is pretomanid, and the second pharmaceutical composition comprises 50 mg to 1000 mg of pretomanid or a pharmaceutically acceptable salt thereof.

In some embodiments, the second therapeutic agent is pretomanid, and the second pharmaceutical composition comprises 50 mg to 800 mg of pretomanid or a pharmaceutically acceptable salt thereof.

In some embodiments, the second therapeutic agent is pretomanid, and the second pharmaceutical composition comprises 50 mg to 600 mg of pretomanid or a pharmaceutically acceptable salt thereof.

In some embodiments, the second therapeutic agent is pretomanid, and the second pharmaceutical composition comprises 100 mg to 800 mg of pretomanid or a pharmaceutically acceptable salt thereof.

In some embodiments, the second therapeutic agent is pretomanid, and the second pharmaceutical composition comprises 100 mg to 600 mg of pretomanid or a pharmaceutically acceptable salt thereof.

In some embodiments, the second therapeutic agent is pretomanid, and the second pharmaceutical composition comprises 150 mg to 600 mg of pretomanid or a pharmaceutically acceptable salt thereof.

In some embodiments, the second therapeutic agent is pretomanid, and the second pharmaceutical composition comprises 200 mg to 600 mg of pretomanid or a pharmaceutically acceptable salt thereof.

In some embodiments, the second therapeutic agent is pretomanid, and the second pharmaceutical composition comprises 100 mg, 200 mg, 300 mg, 400 mg, 500 mg, or 600 mg of pretomanid or a pharmaceutically acceptable salt thereof.

In some embodiments, the third pharmaceutical composition comprises 50 mg to 2000 mg of linezolid or a pharmaceutically acceptable salt thereof.

In some embodiments, the third pharmaceutical composition comprises 10 mg to 1200 mg of linezolid or a pharmaceutically acceptable salt thereof.

In some embodiments, the third pharmaceutical composition comprises 50 mg to 1200 mg of linezolid or a pharmaceutically acceptable salt thereof.

In some embodiments, the third pharmaceutical composition comprises 100 mg to 1200 mg of linezolid or a pharmaceutically acceptable salt thereof.

In some embodiments, the third pharmaceutical composition comprises 200 mg to 1200 mg of linezolid or a pharmaceutically acceptable salt thereof.

In some embodiments, the third pharmaceutical composition comprises 10 mg to 1000 mg of linezolid or a pharmaceutically acceptable salt thereof.

In some embodiments, the third pharmaceutical composition comprises 50 mg to 1000 mg of linezolid or a pharmaceutically acceptable salt thereof.

In some embodiments, the third pharmaceutical composition comprises 100 mg to 1000 mg of linezolid or a pharmaceutically acceptable salt thereof.

In some embodiments, the third pharmaceutical composition comprises 150 mg to 1000 mg of linezolid or a pharmaceutically acceptable salt thereof.

In some embodiments, the third pharmaceutical composition comprises 150 mg to 800 mg of linezolid or a pharmaceutically acceptable salt thereof.

In some embodiments, the third pharmaceutical composition comprises 150 mg to 600 mg of linezolid or a

pharmaceutically acceptable salt thereof.

In some embodiments, the third pharmaceutical composition comprises 200 mg to 600 mg of linezolid or a pharmaceutically acceptable salt thereof.

In some embodiments, the third pharmaceutical composition comprises 100 mg, 200 mg, 300 mg, 400 mg, 500 mg, 600 mg, 700 mg, 800 mg, 900 mg, 1000 mg, 1200 mg, or 1600 mg of linezolid or a pharmaceutically acceptable salt thereof.

In some embodiments, the pharmaceutical combination comprises 150 mg to 450 mg of the compound of formula (I) or a pharmaceutically acceptable salt thereof, 200 mg to 600 mg of pretomanid or a pharmaceutically acceptable salt thereof, and 200 mg to 1200 mg (preferably 200 mg to 600 mg) of linezolid or a pharmaceutically acceptable salt thereof.

In some embodiments, the pharmaceutical combination comprises: (1) 150 mg, 200 mg, 250 mg, 300 mg, 350 mg, 400 mg, or 450 mg of the compound of formula (I) or a pharmaceutically acceptable salt thereof; (2) 100 mg, 200 mg, 300 mg, 400 mg, 500 mg, or 600 mg of pretomanid or a pharmaceutically acceptable salt thereof; and (3) 200 mg, 300 mg, 400 mg, 500 mg, 600 mg, or 1200 mg of linezolid or a pharmaceutically acceptable salt thereof.

In a seventh aspect, the present disclosure provides a use of the pharmaceutical combination according to the first aspect or the fourth aspect, the pharmaceutical composition A according to the second aspect, the pharmaceutical composition B according to the third aspect, the pharmaceutical composition C according to the fifth aspect, or the pharmaceutical composition D according to the sixth aspect in the manufacture of a medicament for the prevention or treatment of a disease caused by *Mycobacterium tuberculosis.*

In some embodiments, the disease caused by *Mycobacterium tuberculosis* is pulmonary tuberculosis, tuberculous peritonitis, tuberculous pleurisy, or bone tuberculosis; preferably, the disease caused by *Mycobacterium tuberculosis* is pulmonary tuberculosis.

In a eighth aspect, the present disclosure provides a use of substance X in the manufacture of a medicament for the prevention or treatment of a disease caused by *Mycobacterium tuberculosis,* the substance X being compound I or a pharmaceutically acceptable salt thereof, wherein the substance X is used in combination with substance Y, wherein the substance Y is a second therapeutic agent or a pharmaceutically acceptable salt thereof, and the second therapeutic agent is pyrazinamide, linezolid, moxifloxacin, pretomanid, or delamanid;
the structure of compound I is shown below:

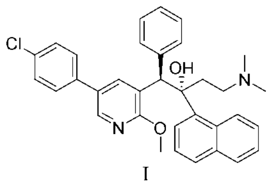

I .

In some embodiments, the second therapeutic agent is pyrazinamide or delamanid.

In some embodiments, the disease caused by *Mycobacterium tuberculosis* is pulmonary tuberculosis, tuberculous peritonitis, tuberculous pleurisy, or bone tuberculosis; preferably, the disease caused by *Mycobacterium tuberculosis* is pulmonary tuberculosis.

In a ninth aspect, the present disclosure provides a use of substance X in the manufacture of a medicament for the prevention or treatment of a disease caused by *Mycobacterium tuberculosis,* the substance X being compound I or a pharmaceutically acceptable salt thereof, wherein the substance X is used in combination with substance Y and substance Z, wherein the substance Y is a second therapeutic agent or a pharmaceutically acceptable salt thereof, and the second therapeutic agent is pretomanid or delamanid; the substance Z is linezolid or a pharmaceutically acceptable salt thereof; the structure of compound I is shown below:

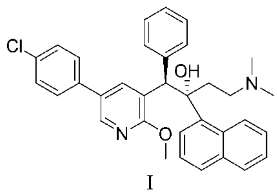

I .

In some embodiments, the second therapeutic agent is pretomanid.

In some embodiments, the disease caused by *Mycobacterium tuberculosis* is pulmonary tuberculosis, tuberculous peritonitis, tuberculous pleurisy, or bone tuberculosis; preferably, the disease caused by *Mycobacterium tuberculosis* is pulmonary tuberculosis.

In a tenth aspect, the present disclosure provides a pharmaceutical combination for use in the prevention and treatment of a disease caused by *Mycobacterium tuberculosis,* wherein the pharmaceutical combination is as defined in the first aspect or the fourth aspect.

In some embodiments, the disease caused by *Mycobacterium tuberculosis* is pulmonary tuberculosis, tuberculous peritonitis, tuberculous pleurisy, or bone tuberculosis; preferably, the disease caused by *Mycobacterium tuberculosis* is pulmonary tuberculosis.

In a eleventh aspect, the present disclosure provides a pharmaceutical composition for use in the prevention and treatment of a disease caused by *Mycobacterium tuberculosis,* wherein the pharmaceutical composition is the pharmaceutical composition A according to the second aspect, the pharmaceutical composition B according to the third aspect, the pharmaceutical composition C according to the fifth aspect, or the pharmaceutical composition D according to the sixth aspect.

In some embodiments, the disease caused by *Mycobacterium tuberculosis* is pulmonary tuberculosis, tuberculous peritonitis, tuberculous pleurisy, or bone tuberculosis; preferably, the disease caused by *Mycobacterium tuberculosis* is pulmonary tuberculosis.

In a twelfth aspect, the present disclosure provides a method for preventing and/or treating a disease caused by *Mycobacterium tuberculosis,* comprising administering to a subject in need thereof an effective amount of the pharmaceutical combination according to the first aspect or the fourth aspect, or administering to a subject in need thereof an effective amount of the pharmaceutical composition A according to the second aspect, the pharmaceutical composition B according to the third aspect, the pharmaceutical composition C according to the fifth aspect, or the pharmaceutical composition D according to the sixth aspect.

In some embodiments, an effective amount of the pharmaceutical combination according to the fourth aspect, the pharmaceutical composition C according to the fifth aspect, or the pharmaceutical composition D according to the sixth aspect is administered to the subject in need thereof.

In some embodiments, the administration regimens (including administration routes, administration doses, administration intervals, etc.) of substance X, substance Y, and substance Z may be the same or different, and may be adjusted by those skilled in the art as needed to provide the optimal therapeutic effect.

In some embodiments, the substance X, the substance Y, and the substance Z may be administered simultaneously or separately.

In some embodiments, the substance X may be administered by any suitable route in the art, including oral administration, injection (e.g., intravenous, intramuscular, subcutaneous), and the like.

In some embodiments, the substance X is administered orally.

In some embodiments, the substance X is administered to the subject at a fixed dose, i.e., a fixed or predetermined amount of dose is administered to the subject. Non-limiting examples of the range of the fixed dose (referring to a single dose) may be 10 mg to 2000 mg, preferably 50 mg to 1000 mg, more preferably 100 mg to 1000 mg, further preferably 100 mg to 800 mg, still further preferably 150 mg to 800 mg, even further preferably 150 mg to 500 mg, and yet even further preferably 150 mg to 450 mg.

In some embodiments, the substance X is administered to the subject at a fixed dose, i.e., a fixed or predetermined amount of dose is administered to the subject. Non-limiting examples of the fixed dose (referring to a single dose) may be, for example, 10 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, or 2000 mg.

In some embodiments, the substance Y may be administered by any suitable route in the art, including oral administration, injection (e.g., intravenous, intramuscular, subcutaneous), and the like.

In some embodiments, the substance Y is administered orally.

In some embodiments, the substance Y is administered to the subject at a fixed dose, i.e., a fixed or predetermined amount of dose is administered to the subject. Non-limiting examples of the range of the fixed dose (referring to a single dose) may be 10 mg to 2000 mg, preferably 50 mg to 1000 mg, more preferably 100 mg to 1000 mg, further preferably 100 mg to 800 mg, still further preferably 150 mg to 800 mg, even further preferably 150 mg to 500 mg, and yet even further preferably 150 mg to 450 mg.

In some embodiments, the substance Y is administered to the subject at a fixed dose, i.e., a fixed or predetermined amount of dose is administered to the subject. Non-limiting examples of the fixed dose (referring to a single dose) may be, for example, 10 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, or 2000 mg.

In some embodiments, the substance Z may be administered by any suitable route in the art, including oral administration, injection (e.g., intravenous, intramuscular, subcutaneous), and the like.

In some embodiments, the substance Z is administered orally.

In some embodiments, the substance Z is administered to the subject at a fixed dose, i.e., a fixed or predetermined amount of dose is administered to the subject. Non-limiting examples of the range of the fixed dose (referring to a single dose) may be 10 mg to 2000 mg, preferably 50 mg to 1000 mg, more preferably 100 mg to 1000 mg, further preferably 100 mg to 800 mg, still further preferably 150 mg to 800 mg, even further preferably 150 mg to 500 mg, and yet even further preferably 150 mg to 450 mg.

In some embodiments, the substance Z is administered to the subject at a fixed dose, i.e., a fixed or predetermined amount of dose is administered to the subject. Non-limiting examples of the fixed dose (referring to a single dose) may be, for example, 10 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, or 2000 mg.

In some embodiments, the substance Y is pretomanid or a pharmaceutically acceptable salt thereof, and the substance Z is linezolid or a pharmaceutically acceptable salt thereof; the substance X is administered to the subject at a fixed dose (referring to a single dose) of 150 mg, the substance Y is administered to the subject at a fixed dose (referring to a single dose) of 100 mg, 200 mg, 300 mg, 400 mg, 500 mg, 600 mg, or 800 mg, and the substance Z is administered to the subject at a fixed dose (referring to a single dose) of 300 mg, 400 mg, 500 mg, 600 mg, 800 mg, 900 mg, 1000 mg, 1100 mg, or 1200 mg.

In some embodiments, the substance Y is pretomanid or a pharmaceutically acceptable salt thereof, and the substance Z is linezolid or a pharmaceutically acceptable salt thereof; the substance X is administered to the subject at a fixed dose (referring to a single dose) of 300 mg, the substance Y is administered to the subject at a fixed dose (referring to a single dose) of 100 mg, 200 mg, 300 mg, 400 mg, 500 mg, 600 mg, or 800 mg, and the substance Z is administered to the subject at a fixed dose (referring to a single dose) of 300 mg, 400 mg, 500 mg, 600 mg, 800 mg, 900 mg, 1000 mg, 1100 mg, or 1200 mg.

In some embodiments, the substance Y is pretomanid or a pharmaceutically acceptable salt thereof, and the substance Z is linezolid or a pharmaceutically acceptable salt thereof; the substance X is administered to the subject at a fixed dose (referring to a single dose) of 450 mg, the substance Y is administered to the subject at a fixed dose (referring to a single dose) of 100 mg, 200 mg, 300 mg, 400 mg, 500 mg, 600 mg, or 800 mg, and the substance Z is administered to the subject at a fixed dose (referring to a single dose) of 300 mg, 400 mg, 500 mg, 600 mg, 800 mg, 900 mg, 1000 mg, 1100 mg, or 1200 mg.

In some embodiments, the aforementioned fixed dose of the substance X may be administered at a frequency of once a day, every other day, three times a week, twice a week, or once a week.

In some embodiments, the substance X is administered at a frequency of once a day.

In some embodiments, the substance X is administered orally according to the aforementioned fixed dose and frequency.

In some embodiments, the aforementioned fixed dose of the substance Y may be administered at a frequency of once a day, every other day, three times a week, twice a week, or once a week.

In some embodiments, the substance Y is administered at a frequency of once a day.

In some embodiments, the substance Y is administered orally according to the aforementioned fixed dose and frequency.

In some embodiments, the aforementioned fixed dose of the substance Z may be administered at a frequency of once a day, every other day, three times a week, twice a week, or once a week.

In some embodiments, the substance Z is administered at a frequency of once a day.

In some embodiments, the substance Z is administered orally according to the aforementioned fixed dose and frequency.

In a thirteenth aspect, the present disclosure provides a pharmaceutical combination comprising:

i) substance X, wherein the substance X is compound I or a pharmaceutically acceptable salt thereof;
ii) substance Y, wherein the substance Y is a second therapeutic agent or a pharmaceutically acceptable salt thereof, and the second therapeutic agent is pyrazinamide, linezolid, moxifloxacin, pretomanid, or delamanid;
iii) one or more pharmaceutically acceptable carriers or media;

optionally, further comprising iv) substance Z, wherein the substance Z is a third therapeutic agent or a pharmaceutically acceptable salt thereof, and the third therapeutic agent is pyrazinamide, linezolid, moxifloxacin, pretomanid, or delamanid;
the structure of compound I is shown below:

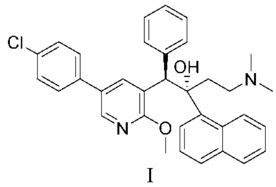

I ;

provided that when substance Z is comprised, the second therapeutic agent and the third therapeutic agent are different.

In some embodiments, the substance X is compound I.

In some embodiments, the substance X is a pharmaceutically acceptable salt of compound I.

In some embodiments, the pharmaceutical combination comprises the substance X, the substance Y, and one or more pharmaceutically acceptable carriers or media; preferably, the pharmaceutical combination consists of the substance X, the substance Y, and one or more pharmaceutically acceptable carriers or media.

In some embodiments, the pharmaceutical combination is a single pharmaceutical composition A1.

In some embodiments, the pharmaceutical composition A1 may be prepared into various suitable dosage forms depending on different administration routes, including enteral dosage forms (e.g., oral dosage forms) and parenteral dosage forms (e.g., injectable dosage forms).

In some embodiments, the pharmaceutical composition A1 is presented in an oral dosage form.

In some embodiments, the pharmaceutical composition A1 is presented in an injectable dosage form.

In some embodiments, the pharmaceutical combination is a pharmaceutical composition B1 comprising a first pharmaceutical composition and a second pharmaceutical composition; the first pharmaceutical composition comprises substance X and a pharmaceutically acceptable carrier or medium; and, the second pharmaceutical composition comprises substance Y and a pharmaceutically acceptable carrier or medium.

In some embodiments, the first pharmaceutical composition is a separate pharmaceutical composition; and/or the second pharmaceutical composition is a separate pharmaceutical composition.

In some embodiments, the pharmaceutical composition B1 consists of the first pharmaceutical composition and the second pharmaceutical composition.

In some embodiments, the first pharmaceutical composition is presented in an oral dosage form or an injectable (e.g., intravenous, subcutaneous, or intramuscular injection) dosage form.

In some embodiments, the second pharmaceutical composition is presented in an oral dosage form or an injectable (e.g., intravenous, subcutaneous, or intramuscular injection) dosage form.

In some embodiments, the first pharmaceutical composition is presented in an oral dosage form, and the second pharmaceutical composition is presented in an oral dosage form.

In some embodiments, the second therapeutic agent is selected from pyrazinamide, moxifloxacin, and pretomanid; preferably, the second therapeutic agent is pyrazinamide.

In some embodiments, the pharmaceutical combination comprises the substance X, the substance Y, the substance Z, and one or more pharmaceutically acceptable carriers or media; preferably, the pharmaceutical composition consists of the substance X, the substance Y, the substance Z, and one or more pharmaceutically acceptable carriers or media.

In some embodiments, the substance X is compound I.

In some embodiments, the substance X is a pharmaceutically acceptable salt of compound I.

In some embodiments, the pharmaceutical combination is a single pharmaceutical composition A2.

In some embodiments, the pharmaceutical composition A2 may be prepared into various suitable dosage forms depending on different administration routes, including enteral dosage forms (e.g., oral dosage forms) and parenteral dosage forms (e.g., injectable dosage forms).

In some embodiments, the pharmaceutical composition A2 is presented in an oral dosage form.

In some embodiments, the pharmaceutical composition A2 is presented in an injectable dosage form.

In some embodiments, the pharmaceutical combination is a pharmaceutical composition C1 comprising a third pharmaceutical composition, a fourth pharmaceutical composition, and a fifth pharmaceutical composition; the third pharmaceutical composition comprises substance X and a pharmaceutically acceptable carrier or medium; the fourth pharmaceutical composition comprises substance Y and a pharmaceutically acceptable carrier or medium; and/or the fifth pharmaceutical composition comprises substance Z and a pharmaceutically acceptable carrier or medium.

In some embodiments, the third pharmaceutical composition is a separate pharmaceutical composition; the fourth pharmaceutical composition is a separate pharmaceutical composition; and/or the fifth pharmaceutical composition is a separate pharmaceutical composition.

In some embodiments, the pharmaceutical composition C1 consists of the third pharmaceutical composition, the fourth pharmaceutical composition, and the fifth pharmaceutical composition.

In some embodiments, the third pharmaceutical composition is presented in an oral dosage form or an injectable (e.g., intravenous, subcutaneous, or intramuscular injection) dosage form.

In some embodiments, the fourth pharmaceutical composition is presented in an oral dosage form or an injectable (e.g., intravenous, subcutaneous, or intramuscular injection) dosage form.

In some embodiments, the fifth pharmaceutical composition is presented in an oral dosage form or an injectable (e.g., intravenous, subcutaneous, or intramuscular injection) dosage form.

In some embodiments, the third pharmaceutical composition is presented in an oral dosage form, the fourth pharmaceutical composition is presented in an oral dosage form, and the fifth pharmaceutical composition is presented in an oral dosage form.

In some embodiments, the second therapeutic agent is pretomanid or delamanid; and/or the third therapeutic agent is linezolid.

In some embodiments, the pharmaceutical combination is presented in an oral dosage form.

In a fourteenth aspect, the present disclosure provides a pharmaceutical combination comprising:

i) substance X, wherein the substance X is compound I or a pharmaceutically acceptable salt thereof;
ii) substance Y, wherein the substance Y is a second therapeutic agent or a pharmaceutically acceptable salt thereof, and the second therapeutic agent is pyrazinamide, linezolid, moxifloxacin, pretomanid, or delamanid;
iii) one or more pharmaceutically acceptable carriers or media;
the structure of compound I is shown below:

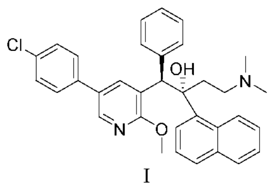

I

.

In some embodiments, the pharmaceutical combination consists of the substance X, the substance Y, and one or more pharmaceutically acceptable carriers or media.

In some embodiments, the pharmaceutical combination is a single pharmaceutical composition A1.

In some embodiments, the pharmaceutical composition A1 may be prepared into various suitable dosage forms depending on different administration routes, including enteral dosage forms (e.g., oral dosage forms) and parenteral dosage forms (e.g., injectable dosage forms).

In some embodiments, the pharmaceutical composition A1 is presented in an oral dosage form.

In some embodiments, the pharmaceutical composition A1 is presented in an injectable dosage form.

In some embodiments, the pharmaceutical combination is a pharmaceutical composition B1 comprising a first pharmaceutical composition and a second pharmaceutical composition; the first pharmaceutical composition comprises substance X and a pharmaceutically acceptable carrier or medium; and, the second pharmaceutical composition comprises substance Y and a pharmaceutically acceptable carrier or medium.

In some embodiments, the first pharmaceutical composition is a separate pharmaceutical composition; and/or the second pharmaceutical composition is a separate pharmaceutical composition.

In some embodiments, the pharmaceutical composition B1 consists of the first pharmaceutical composition and the second pharmaceutical composition.

In some embodiments, the first pharmaceutical composition is presented in an oral dosage form or an injectable (e.g., intravenous, subcutaneous, or intramuscular injection) dosage form.

In some embodiments, the second pharmaceutical composition is presented in an oral dosage form or an injectable (e.g., intravenous, subcutaneous, or intramuscular injection) dosage form.

In some embodiments, the first pharmaceutical composition is presented in an oral dosage form, and the second pharmaceutical composition is presented in an oral dosage form.

In some embodiments, the substance X is compound I.

In some embodiments, the substance X is a pharmaceutically acceptable salt of compound I; preferably, the substance X is a hydrochloride, sulfate, citrate, maleate, or fumarate salt of compound I; more preferably, the substance X is a fumarate salt of compound I.

In some embodiments, the second therapeutic agent is selected from pyrazinamide, moxifloxacin, and pretomanid; preferably, the second therapeutic agent is pyrazinamide.

In some embodiments, the pharmaceutical combination is presented in an oral dosage form.

In some embodiments, the substance X and the substance Y are administered simultaneously or separately.

In some embodiments, the substance X and the substance Y are administered orally.

In some embodiments, the substance X is administered once daily (QD) or twice daily (BID), and/or the substance Y is administered once daily (QD) or twice daily (BID).

In some embodiments, the substance X is administered once weekly, twice weekly, three times weekly, or once every other day, and/or the substance Y is administered once weekly, twice weekly, three times weekly, or once every other day.

In some embodiments, the pharmaceutical combination comprises 10 mg to 2000 mg of the compound of formula (I) or a pharmaceutically acceptable salt thereof.

In some embodiments, the pharmaceutical combination comprises 50 mg to 1000 mg of the compound of formula (I) or a pharmaceutically acceptable salt thereof.

In some embodiments, the pharmaceutical combination comprises 100 mg to 1000 mg of the compound of formula (I) or a pharmaceutically acceptable salt thereof.

In some embodiments, the pharmaceutical combination comprises 100 mg to 800 mg of the compound of formula (I) or a pharmaceutically acceptable salt thereof.

In some embodiments, the pharmaceutical combination comprises 150 mg to 800 mg of the compound of formula (I) or a pharmaceutically acceptable salt thereof.

In some embodiments, the pharmaceutical combination comprises 150 mg to 500 mg of the compound of formula (I) or a pharmaceutically acceptable salt thereof.

In some embodiments, the pharmaceutical combination comprises 150 mg to 450 mg of the compound of formula (I) or a pharmaceutically acceptable salt thereof.

In some embodiments, the pharmaceutical combination comprises 150 mg, 200 mg, 250 mg, 300 mg, 350 mg, 400 mg, or 450 mg of the compound of formula (I) or a pharmaceutically acceptable salt thereof.

In some embodiments, the pharmaceutical combination comprises 450 mg of the compound of formula (I) or a pharmaceutically acceptable salt thereof.

In some embodiments, the pharmaceutical combination comprises 300 mg of the compound of formula (I) or a pharmaceutically acceptable salt thereof.

In some embodiments, the pharmaceutical combination comprises 150 mg of the compound of formula (I) or a pharmaceutically acceptable salt thereof.

In some embodiments, the second therapeutic agent is pyrazinamide, and the pharmaceutical combination comprises 10 mg to 4000 mg of pyrazinamide or a pharmaceutically acceptable salt thereof.

In some embodiments, the second therapeutic agent is pyrazinamide, and the pharmaceutical combination comprises 50 mg to 4000 mg of pyrazinamide or a pharmaceutically acceptable salt thereof.

In some embodiments, the second therapeutic agent is pyrazinamide, and the pharmaceutical combination comprises 100 mg to 4000 mg of pyrazinamide or a pharmaceutically acceptable salt thereof.

In some embodiments, the second therapeutic agent is pyrazinamide, and the pharmaceutical combination comprises 150 mg to 3000 mg of pyrazinamide or a pharmaceutically acceptable salt thereof.

In some embodiments, the second therapeutic agent is pyrazinamide, and the pharmaceutical combination comprises 200 mg to 3000 mg of pyrazinamide or a pharmaceutically acceptable salt thereof.

In some embodiments, the second therapeutic agent is pyrazinamide, and the pharmaceutical combination comprises 250 mg to 3000 mg of pyrazinamide or a pharmaceutically acceptable salt thereof.

In some embodiments, the second therapeutic agent is pyrazinamide, and the pharmaceutical combination comprises 300 mg to 2000 mg of pyrazinamide or a pharmaceutically acceptable salt thereof.

In some embodiments, the second therapeutic agent is pyrazinamide, and the pharmaceutical combination comprises 400 mg to 1500 mg of pyrazinamide or a pharmaceutically acceptable salt thereof.

In some embodiments, the second therapeutic agent is pyrazinamide, and the pharmaceutical combination comprises 400 mg to 1000 mg of pyrazinamide or a pharmaceutically acceptable salt thereof.

In some embodiments, the second therapeutic agent is pyrazinamide, and the pharmaceutical combination comprises 400 mg to 800 mg of pyrazinamide or a pharmaceutically acceptable salt thereof.

In some embodiments, the second therapeutic agent is pyrazinamide, and the pharmaceutical combination comprises 400 mg to 500 mg of pyrazinamide or a pharmaceutically acceptable salt thereof.

In some embodiments, the second therapeutic agent is pyrazinamide, and the pharmaceutical combination comprises 300 mg, 400 mg, 500 mg, 600 mg, or 800 mg of pyrazinamide or a pharmaceutically acceptable salt thereof.

In some embodiments, the second therapeutic agent is delamanid, and the pharmaceutical combination comprises 10 mg to 1000 mg of delamanid or a pharmaceutically acceptable salt thereof.

In some embodiments, the second therapeutic agent is delamanid, and the pharmaceutical combination comprises 25 mg to 1000 mg of delamanid or a pharmaceutically acceptable salt thereof.

In some embodiments, the second therapeutic agent is delamanid, and the pharmaceutical combination

comprises 50 mg to 1000 mg of delamanid or a pharmaceutically acceptable salt thereof.

In some embodiments, the second therapeutic agent is delamanid, and the pharmaceutical combination comprises 50 mg to 800 mg of delamanid or a pharmaceutically acceptable salt thereof.

In some embodiments, the second therapeutic agent is delamanid, and the pharmaceutical combination comprises 50 mg to 600 mg of delamanid or a pharmaceutically acceptable salt thereof.

In some embodiments, the second therapeutic agent is delamanid, and the pharmaceutical combination comprises 100 mg to 800 mg of delamanid or a pharmaceutically acceptable salt thereof.

In some embodiments, the second therapeutic agent is delamanid, and the pharmaceutical combination comprises 100 mg to 600 mg of delamanid or a pharmaceutically acceptable salt thereof.

In some embodiments, the second therapeutic agent is delamanid, and the pharmaceutical combination comprises 150 mg to 600 mg of delamanid or a pharmaceutically acceptable salt thereof.

In some embodiments, the second therapeutic agent is delamanid, and the pharmaceutical combination comprises 200 mg to 600 mg of delamanid or a pharmaceutically acceptable salt thereof.

In some embodiments, the second therapeutic agent is delamanid, and the pharmaceutical combination comprises 100 mg, 200 mg, 300 mg, 400 mg, 500 mg, or 600 mg of delamanid or a pharmaceutically acceptable salt thereof.

In some embodiments, the second therapeutic agent is pretomanid, and the pharmaceutical combination comprises 10 mg to 1000 mg of pretomanid or a pharmaceutically acceptable salt thereof.

In some embodiments, the second therapeutic agent is pretomanid, and the pharmaceutical combination comprises 25 mg to 1000 mg of pretomanid or a pharmaceutically acceptable salt thereof.

In some embodiments, the second therapeutic agent is pretomanid, and the pharmaceutical combination comprises 50 mg to 1000 mg of pretomanid or a pharmaceutically acceptable salt thereof.

In some embodiments, the second therapeutic agent is pretomanid, and the pharmaceutical combination comprises 50 mg to 800 mg of pretomanid or a pharmaceutically acceptable salt thereof.

In some embodiments, the second therapeutic agent is pretomanid, and the pharmaceutical combination comprises 50 mg to 600 mg of pretomanid or a pharmaceutically acceptable salt thereof.

In some embodiments, the second therapeutic agent is pretomanid, and the pharmaceutical combination comprises 100 mg to 800 mg of pretomanid or a pharmaceutically acceptable salt thereof.

In some embodiments, the second therapeutic agent is pretomanid, and the pharmaceutical combination comprises 100 mg to 600 mg of pretomanid or a pharmaceutically acceptable salt thereof.

In some embodiments, the second therapeutic agent is pretomanid, and the pharmaceutical combination comprises 150 mg to 600 mg of pretomanid or a pharmaceutically acceptable salt thereof.

In some embodiments, the second therapeutic agent is pretomanid, and the pharmaceutical combination comprises 200 mg to 600 mg of pretomanid or a pharmaceutically acceptable salt thereof.

In some embodiments, the second therapeutic agent is pretomanid, and the pharmaceutical combination comprises 100 mg, 200 mg, 300 mg, 400 mg, 500 mg, or 600 mg of pretomanid or a pharmaceutically acceptable salt thereof.

In some embodiments, the pharmaceutical combination comprises 150 mg, 200 mg, 250 mg, 300 mg, 350 mg, 400 mg, or 450 mg of the compound of formula (I) or a pharmaceutically acceptable salt thereof, and the pharmaceutical combination comprises 300 mg, 400 mg, 500 mg, 600 mg, or 800 mg of pyrazinamide or a pharmaceutically acceptable salt thereof.

In some embodiments, the pharmaceutical combination comprises 150 mg, 300 mg, or 450 mg of the compound of formula (I) or a pharmaceutically acceptable salt thereof, and the pharmaceutical combination comprises 300 mg, 400 mg, 500 mg, 600 mg, or 800 mg of pyrazinamide or a pharmaceutically acceptable salt thereof.

In a fifteenth aspect, the present disclosure provides a pharmaceutical combination comprising:

i) substance X, wherein the substance X is compound I or a pharmaceutically acceptable salt thereof;
ii) substance Y, wherein the substance Y is a second therapeutic agent or a pharmaceutically acceptable salt thereof, and the second therapeutic agent is pyrazinamide, linezolid, moxifloxacin, pretomanid, or delamanid;
iii) one or more pharmaceutically acceptable carriers or media;
iv) substance Z, wherein the substance Z is a third therapeutic agent or a pharmaceutically acceptable salt thereof, and the third therapeutic agent is pyrazinamide, linezolid, moxifloxacin, pretomanid, or delamanid;

the structure of compound I is shown below:

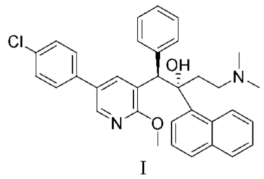

I

;

provided that the second therapeutic agent and the third therapeutic agent are different.

In some embodiments, the pharmaceutical combination consists of the substance X, the substance Y, the substance Z, and one or more pharmaceutically acceptable carriers or media.

In some embodiments, the pharmaceutical combination is a single pharmaceutical composition A2.

In some embodiments, the pharmaceutical composition A2 may be prepared into various suitable dosage forms depending on different administration routes, including enteral dosage forms (e.g., oral dosage forms) and parenteral dosage forms (e.g., injectable dosage forms).

In some embodiments, the pharmaceutical composition A2 is presented in an oral dosage form.

In some embodiments, the pharmaceutical composition A2 is presented in an injectable dosage form.

In some embodiments, the pharmaceutical combination is a pharmaceutical composition C1 comprising a third pharmaceutical composition, a fourth pharmaceutical composition, and a fifth pharmaceutical composition; the third pharmaceutical composition comprises substance X and a pharmaceutically acceptable carrier or medium; the fourth pharmaceutical composition comprises substance Y and a pharmaceutically acceptable carrier or medium; and/or the fifth pharmaceutical composition comprises substance Z and a pharmaceutically acceptable carrier or medium.

In some embodiments, the third pharmaceutical composition is a separate pharmaceutical composition; the fourth pharmaceutical composition is a separate pharmaceutical composition; and/or the fifth pharmaceutical composition is a separate pharmaceutical composition.

In some embodiments, the pharmaceutical composition C1 consists of the third pharmaceutical composition, the fourth pharmaceutical composition, and the fifth pharmaceutical composition.

In some embodiments, the third pharmaceutical composition is presented in an oral dosage form or an injectable (e.g., intravenous, subcutaneous, or intramuscular injection) dosage form.

In some embodiments, the fourth pharmaceutical composition is presented in an oral dosage form or an injectable (e.g., intravenous, subcutaneous, or intramuscular injection) dosage form.

In some embodiments, the fifth pharmaceutical composition is presented in an oral dosage form or an injectable (e.g., intravenous, subcutaneous, or intramuscular injection) dosage form.

In some embodiments, the third pharmaceutical composition is presented in an oral dosage form, the fourth pharmaceutical composition is presented in an oral dosage form, and the fifth pharmaceutical composition is presented in an oral dosage form.

In some embodiments, the substance X is compound I.

In some embodiments, the substance X is a pharmaceutically acceptable salt of compound I.

In some embodiments, the second therapeutic agent is pretomanid or delamanid; and/or the third therapeutic agent is linezolid.

In some embodiments, the second therapeutic agent is pretomanid and the third therapeutic agent is linezolid; alternatively, the second therapeutic agent delamanid and the third therapeutic agent is linezolid.

In some embodiments, the pharmaceutical combination is presented in an oral dosage form.

In some embodiments, the substance X, the substance Y, and the substance Z are administered simultaneously or separately.

In some embodiments, the substance X, the substance Y, and the substance Z are administered orally.

In some embodiments, the pharmaceutical combination comprises 10 mg to 2000 mg of the compound of formula (I) or a pharmaceutically acceptable salt thereof.

In some embodiments, the pharmaceutical combination comprises 50 mg to 1000 mg of the compound of formula (I) or a pharmaceutically acceptable salt thereof.

In some embodiments, the pharmaceutical combination comprises 100 mg to 1000 mg of the compound of formula (I) or a pharmaceutically acceptable salt thereof.

In some embodiments, the pharmaceutical combination comprises 100 mg to 800 mg of the compound of formula (I) or a pharmaceutically acceptable salt thereof.

In some embodiments, the pharmaceutical combination comprises 150 mg to 800 mg of the compound of formula (I) or a pharmaceutically acceptable salt thereof.

In some embodiments, the pharmaceutical combination comprises 150 mg to 500 mg of the compound of formula (I) or a pharmaceutically acceptable salt thereof.

In some embodiments, the pharmaceutical combination comprises 150 mg to 450 mg of the compound of formula (I) or a pharmaceutically acceptable salt thereof.

In some embodiments, the pharmaceutical combination comprises 150 mg, 200 mg, 250 mg, 300 mg, 350 mg, 400 mg, or 450 mg of the compound of formula (I) or a pharmaceutically acceptable salt thereof.

In some embodiments, the pharmaceutical combination comprises 450 mg of the compound of formula (I) or a pharmaceutically acceptable salt thereof.

In some embodiments, the pharmaceutical combination comprises 300 mg of the compound of formula (I) or a pharmaceutically acceptable salt thereof.

In some embodiments, the pharmaceutical combination comprises 150 mg of the compound of formula (I) or a pharmaceutically acceptable salt thereof.

In some embodiments, the second therapeutic agent is delamanid, and the pharmaceutical combination comprises 10 mg to 1000 mg of delamanid or a pharmaceutically acceptable salt thereof.

In some embodiments, the second therapeutic agent is delamanid, and the pharmaceutical combination comprises 25 mg to 1000 mg of delamanid or a pharmaceutically acceptable salt thereof.

In some embodiments, the second therapeutic agent is delamanid, and the pharmaceutical combination comprises 50 mg to 1000 mg of delamanid or a pharmaceutically acceptable salt thereof.

In some embodiments, the second therapeutic agent is delamanid, and the pharmaceutical combination comprises 50 mg to 800 mg of delamanid or a pharmaceutically acceptable salt thereof.

In some embodiments, the second therapeutic agent is delamanid, and the pharmaceutical combination comprises 50 mg to 600 mg of delamanid or a pharmaceutically acceptable salt thereof.

In some embodiments, the second therapeutic agent is delamanid, and the pharmaceutical combination comprises 100 mg to 800 mg of delamanid or a pharmaceutically acceptable salt thereof.

In some embodiments, the second therapeutic agent is delamanid, and the pharmaceutical combination comprises 100 mg to 600 mg of delamanid or a pharmaceutically acceptable salt thereof.

In some embodiments, the second therapeutic agent is delamanid, and the pharmaceutical combination comprises 150 mg to 600 mg of delamanid or a pharmaceutically acceptable salt thereof.

In some embodiments, the second therapeutic agent is delamanid, and the pharmaceutical combination comprises 200 mg to 600 mg of delamanid or a pharmaceutically acceptable salt thereof.

In some embodiments, the second therapeutic agent is delamanid, and the pharmaceutical combination comprises 100 mg, 200 mg, 300 mg, 400 mg, 500 mg, or 600 mg of delamanid or a pharmaceutically acceptable salt thereof.

In some embodiments, the second therapeutic agent is pretomanid, and the pharmaceutical combination comprises 10 mg to 1000 mg of pretomanid or a pharmaceutically acceptable salt thereof.

In some embodiments, the second therapeutic agent is pretomanid, and the pharmaceutical combination comprises 25 mg to 1000 mg of pretomanid or a pharmaceutically acceptable salt thereof.

In some embodiments, the second therapeutic agent is pretomanid, and the pharmaceutical combination comprises 50 mg to 1000 mg of pretomanid or a pharmaceutically acceptable salt thereof.

In some embodiments, the second therapeutic agent is pretomanid, and the pharmaceutical combination comprises 50 mg to 800 mg of pretomanid or a pharmaceutically acceptable salt thereof.

In some embodiments, the second therapeutic agent is pretomanid, and the pharmaceutical combination comprises 50 mg to 600 mg of pretomanid or a pharmaceutically acceptable salt thereof.

In some embodiments, the second therapeutic agent is pretomanid, and the pharmaceutical combination comprises 100 mg to 800 mg of pretomanid or a pharmaceutically acceptable salt thereof.

In some embodiments, the second therapeutic agent is pretomanid, and the pharmaceutical combination comprises 100 mg to 600 mg of pretomanid or a pharmaceutically acceptable salt thereof.

In some embodiments, the second therapeutic agent is pretomanid, and the pharmaceutical combination comprises 150 mg to 600 mg of pretomanid or a pharmaceutically acceptable salt thereof.

In some embodiments, the second therapeutic agent is pretomanid, and the pharmaceutical combination comprises 200 mg to 600 mg of pretomanid or a pharmaceutically acceptable salt thereof.

In some embodiments, the second therapeutic agent is pretomanid, and the pharmaceutical combination comprises 100 mg, 200 mg, 300 mg, 400 mg, 500 mg, or 600 mg of pretomanid or a pharmaceutically acceptable salt thereof.

In some embodiments, the third therapeutic agent is linezolid, and the pharmaceutical combination comprises 10 mg to 2000 mg of linezolid or a pharmaceutically acceptable salt thereof.

In some embodiments, the third therapeutic agent is linezolid, and the pharmaceutical combination comprises 50 mg to 2000 mg of linezolid or a pharmaceutically acceptable salt thereof.

In some embodiments, the third therapeutic agent is linezolid, and the pharmaceutical combination comprises 10 mg to 1200 mg of linezolid or a pharmaceutically acceptable salt thereof.

In some embodiments, the third therapeutic agent is linezolid, and the pharmaceutical combination comprises 50 mg to 1200 mg of linezolid or a pharmaceutically acceptable salt thereof.
In some embodiments, the third therapeutic agent is linezolid, and the pharmaceutical combination comprises 100 mg to 1200 mg of linezolid or a pharmaceutically acceptable salt thereof.
In some embodiments, the third therapeutic agent is linezolid, and the pharmaceutical combination comprises 200 mg to 1200 mg of linezolid or a pharmaceutically acceptable salt thereof.
In some embodiments, the third therapeutic agent is linezolid, and the pharmaceutical combination comprises 10 mg to 1000 mg of linezolid or a pharmaceutically acceptable salt thereof.
In some embodiments, the third therapeutic agent is linezolid, and the pharmaceutical combination comprises 50 mg to 1000 mg of linezolid or a pharmaceutically acceptable salt thereof.
In some embodiments, the third therapeutic agent is linezolid, and the pharmaceutical combination comprises 100 mg to 1000 mg of linezolid or a pharmaceutically acceptable salt thereof.
In some embodiments, the third therapeutic agent is linezolid, and the pharmaceutical combination comprises 150 mg to 1000 mg of linezolid or a pharmaceutically acceptable salt thereof.
In some embodiments, the third therapeutic agent is linezolid, and the pharmaceutical combination comprises 150 mg to 800 mg of linezolid or a pharmaceutically acceptable salt thereof.
In some embodiments, the third therapeutic agent is linezolid, and the pharmaceutical combination comprises 150 mg to 600 mg of linezolid or a pharmaceutically acceptable salt thereof.
In some embodiments, the third therapeutic agent is linezolid, and the pharmaceutical combination comprises 200 mg to 600 mg of linezolid or a pharmaceutically acceptable salt thereof.
In some embodiments, the third therapeutic agent is linezolid, and the third pharmaceutical combination comprises 100 mg, 200 mg, 300 mg, 400 mg, 500 mg, 600 mg, 700 mg, 800 mg, 900 mg, 1000 mg, 1200 mg, or 1600 mg of linezolid or a pharmaceutically acceptable salt thereof.
In some embodiments, the pharmaceutical combination comprises 150 mg to 450 mg of the compound of formula (I) or a pharmaceutically acceptable salt thereof, 200 mg to 600 mg of pretomanid or a pharmaceutically acceptable salt thereof, and 200 mg to 1200 mg (preferably 200 mg to 600 mg) of linezolid or a pharmaceutically acceptable salt thereof.
In some embodiments, the pharmaceutical combination comprises 150 mg to 450 mg of the compound of formula (I) or a pharmaceutically acceptable salt thereof, 200 mg to 600 mg of delamanid or a pharmaceutically acceptable salt thereof, and 200 mg to 1200 mg (preferably 200 mg to 600 mg) of linezolid or a pharmaceutically acceptable salt thereof.
In some embodiments, the pharmaceutical combination comprises: (1) 150 mg, 200 mg, 250 mg, 300 mg, 350 mg, 400 mg, or 450 mg of the compound of formula (I) or a pharmaceutically acceptable salt thereof; (2) 100 mg, 200 mg, 300 mg, 400 mg, 500 mg, or 600 mg of pretomanid or a pharmaceutically acceptable salt thereof; and (3) 200 mg, 300 mg, 400 mg, 500 mg, 600 mg, or 1200 mg of linezolid or a pharmaceutically acceptable salt thereof.
In some embodiments, the pharmaceutical combination comprises: (1) 150 mg, 200 mg, 250 mg, 300 mg, 350 mg, 400 mg, or 450 mg of the compound of formula (I) or a pharmaceutically acceptable salt thereof; (2) 100 mg, 200 mg, 300 mg, 400 mg, 500 mg, or 600 mg of delamanid or a pharmaceutically acceptable salt thereof; and (3) 200 mg, 300 mg, 400 mg, 500 mg, 600 mg, or 1200 mg of linezolid or a pharmaceutically acceptable salt thereof.
In a sixteenth aspect, the present disclosure provides a use of the pharmaceutical composition according to any one of the thirteenth aspect to the fifteenth aspect in the manufacture of a medicament for the prevention and/or treatment of a disease caused by *Mycobacterium tuberculosis.*
In some embodiments, the disease caused by *Mycobacterium tuberculosis* is pulmonary tuberculosis, tuberculous peritonitis, tuberculous pleurisy, or bone tuberculosis; preferably, the disease caused by *Mycobacterium tuberculosis* is pulmonary tuberculosis.
In a seventeenth aspect, the present disclosure provides a pharmaceutical combination for use in the prevention and treatment of a disease caused by *Mycobacterium tuberculosis,* wherein the pharmaceutical combination is as defined in the thirteenth aspect, the fourteenth aspect, or the fifteenth aspect.
In some embodiments, the disease caused by *Mycobacterium tuberculosis* is pulmonary tuberculosis, tuberculous peritonitis, tuberculous pleurisy, or bone tuberculosis; preferably, the disease caused by *Mycobacterium tuberculosis* is pulmonary tuberculosis.
In a eighteenth aspect, the present disclosure provides a method for preventing and/or treating a disease caused by *Mycobacterium tuberculosis,* comprising administering to a subject in need thereof an effective amount of the pharmaceutical combination according to any one of the thirteenth aspect to the fifteenth aspect.
In some embodiments, an effective amount of the pharmaceutical combination according to the thirteenth aspect is administered to a subject in need thereof.
In some embodiments, an effective amount of the pharmaceutical combination according to the fourteenth aspect is administered to a subject in need thereof.
In some embodiments, the substance Y is pyrazinamide or a pharmaceutically acceptable salt thereof.
In some embodiments, the substance Y is moxifloxacin or a pharmaceutically acceptable salt thereof.

In some embodiments, the substance Y is delamanid or a pharmaceutically acceptable salt thereof.

In some embodiments, the substance Y is linezolid or a pharmaceutically acceptable salt thereof.

In some embodiments, the substance Y is pretomanid or a pharmaceutically acceptable salt thereof.

In some embodiments, the administration regimens (including administration routes, administration doses, administration intervals, etc.) of the substance X and the substance Y may be the same or different, and may be adjusted by those skilled in the art as needed to provide the optimal therapeutic effect.

In some embodiments, the substance X and the substance Y may be administered simultaneously or separately.

In some embodiments, the substance X may be administered by any suitable route in the art, including oral administration, injection (e.g., intravenous, intramuscular, subcutaneous), and the like.

In some embodiments, the substance X is administered orally.

In some embodiments, the substance X is administered to the subject at a fixed dose, i.e., a fixed or predetermined amount of dose is administered to the subject. Non-limiting examples of the range of the fixed dose (referring to a single dose) may be 10 mg to 2000 mg, preferably 50 mg to 1000 mg, more preferably 100 mg to 1000 mg, further preferably 100 mg to 800 mg, still further preferably 150 mg to 800 mg, even further preferably 150 mg to 500 mg, and yet even further preferably 150 mg to 450 mg.

In some embodiments, the substance X is administered to the subject at a fixed dose, i.e., a fixed or predetermined amount of dose is administered to the subject. Non-limiting examples of the fixed dose (referring to a single dose) may be, for example, 10 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, or 2000 mg.

In some embodiments, the substance Y may be administered by any suitable route in the art, including oral administration, injection (e.g., intravenous, intramuscular, subcutaneous), and the like.

In some embodiments, the substance Y is administered orally.

In some embodiments, the substance Y is administered to the subject at a fixed dose, i.e., a fixed or predetermined amount of dose is administered to the subject. Non-limiting examples of the range of the fixed dose (referring to a single dose) may be 10 mg to 2000 mg, preferably 50 mg to 1000 mg, more preferably 100 mg to 1000 mg, further preferably 100 mg to 800 mg, still further preferably 150 mg to 800 mg, even further preferably 150 mg to 500 mg, and yet even further preferably 150 mg to 450 mg.

In some embodiments, the substance Y is administered to the subject at a fixed dose, i.e., a fixed or predetermined amount of dose is administered to the subject. Non-limiting examples of the fixed dose (referring to a single dose) may be, for example, 10 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, or 2000 mg.

In some embodiments, the substance X is administered to the subject at a fixed dose (referring to a single dose) of 150 mg, and the substance Y is pyrazinamide or a pharmaceutically acceptable salt thereof, wherein the substance Y is administered to the subject at a fixed dose (referring to a single dose) of 300 mg, 400 mg, 500 mg, 600 mg, or 800 mg.

In some embodiments, the substance X is administered to the subject at a fixed dose (referring to a single dose) of 300 mg, and the substance Y is pyrazinamide or a pharmaceutically acceptable salt thereof, wherein the substance Y is administered to the subject at a fixed dose (referring to a single dose) of 300 mg, 400 mg, 500 mg, 600 mg, or 800 mg.

In some embodiments, the substance X is administered to the subject at a fixed dose (referring to a single dose) of 450 mg, and the substance Y is pyrazinamide or a pharmaceutically acceptable salt thereof, wherein the substance Y is administered to the subject at a fixed dose (referring to a single dose) of 300 mg, 400 mg, 500 mg, 600 mg, or 800 mg.

In some embodiments, the aforementioned fixed dose of the substance X may be administered at a frequency of once a day, every other day, three times a week, twice a week, or once a week.

In some embodiments, the substance X is administered at a frequency of once a day.

In some embodiments, the substance X is administered orally according to the aforementioned fixed dose and frequency.

In some embodiments, the aforementioned fixed dose of the substance Y may be administered at a frequency of once a day, every other day, three times a week, twice a week, or once a week.

In some embodiments, the substance Y is administered at a frequency of once a day.

In some embodiments, the substance Y is administered orally according to the aforementioned fixed dose and frequency.

In some embodiments, an effective amount of the pharmaceutical combination according to the fifteenth aspect is administered to a subject in need thereof.

In some embodiments, the administration regimens (including administration routes, administration doses, administration intervals, etc.) of substance X, substance Y, and substance Z may be the same or different, and may be

adjusted by those skilled in the art as needed to provide the optimal therapeutic effect.

In some embodiments, the substance X, the substance Y, and the substance Z may be administered simultaneously or separately.

In some embodiments, the substance X may be administered by any suitable route in the art, including oral administration, injection (e.g., intravenous, intramuscular, subcutaneous), and the like.

In some embodiments, the substance X is administered orally.

In some embodiments, the substance X is administered to the subject at a fixed dose, i.e., a fixed or predetermined amount of dose is administered to the subject. Non-limiting examples of the range of the fixed dose (referring to a single dose) may be 10 mg to 2000 mg, preferably 50 mg to 1000 mg, more preferably 100 mg to 1000 mg, further preferably 100 mg to 800 mg, still further preferably 150 mg to 800 mg, even further preferably 150 mg to 500 mg, and yet even further preferably 150 mg to 450 mg.

In some embodiments, the substance X is administered to the subject at a fixed dose, i.e., a fixed or predetermined amount of dose is administered to the subject. Non-limiting examples of the fixed dose (referring to a single dose) may be, for example, 10 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, or 2000 mg.

In some embodiments, the substance Y may be administered by any suitable route in the art, including oral administration, injection (e.g., intravenous, intramuscular, subcutaneous), and the like.

In some embodiments, the substance Y is administered orally.

In some embodiments, the substance Y is administered to the subject at a fixed dose, i.e., a fixed or predetermined amount of dose is administered to the subject. Non-limiting examples of the range of the fixed dose (referring to a single dose) may be 10 mg to 2000 mg, preferably 50 mg to 1000 mg, more preferably 100 mg to 1000 mg, further preferably 100 mg to 800 mg, still further preferably 150 mg to 800 mg, even further preferably 150 mg to 500 mg, and yet even further preferably 150 mg to 450 mg.

In some embodiments, the substance Y is administered to the subject at a fixed dose, i.e., a fixed or predetermined amount of dose is administered to the subject. Non-limiting examples of the fixed dose (referring to a single dose) may be, for example, 10 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, or 2000 mg.

In some embodiments, the substance Z may be administered by any suitable route in the art, including oral administration, injection (e.g., intravenous, intramuscular, subcutaneous), and the like.

In some embodiments, the substance Z is administered orally.

In some embodiments, the substance Z is administered to the subject at a fixed dose, i.e., a fixed or predetermined amount of dose is administered to the subject. Non-limiting examples of the range of the fixed dose (referring to a single dose) may be 10 mg to 2000 mg, preferably 50 mg to 1000 mg, more preferably 100 mg to 1000 mg, further preferably 100 mg to 800 mg, still further preferably 150 mg to 800 mg, even further preferably 150 mg to 500 mg, and yet even further preferably 150 mg to 450 mg.

In some embodiments, the substance Z is administered to the subject at a fixed dose, i.e., a fixed or predetermined amount of dose is administered to the subject. Non-limiting examples of the fixed dose (referring to a single dose) may be, for example, 10 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, or 2000 mg.

In some embodiments, the substance Y is pretomanid or a pharmaceutically acceptable salt thereof, and the substance Z is linezolid or a pharmaceutically acceptable salt thereof; the substance X is administered to the subject at a fixed dose (referring to a single dose) of 150 mg, the substance Y is administered to the subject at a fixed dose (referring to a single dose) of 100 mg, 200 mg, 300 mg, 400 mg, 500 mg, 600 mg, or 800 mg, and the substance Z is administered to the subject at a fixed dose (referring to a single dose) of 300 mg, 400 mg, 500 mg, 600 mg, 800 mg, 900 mg, 1000 mg, 1100 mg, or 1200 mg.

In some embodiments, the substance Y is pretomanid or a pharmaceutically acceptable salt thereof, and the substance Z is linezolid or a pharmaceutically acceptable salt thereof; the substance X is administered to the subject at a fixed dose (referring to a single dose) of 300 mg, the substance Y is administered to the subject at a fixed dose (referring to a single dose) of 100 mg, 200 mg, 300 mg, 400 mg, 500 mg, 600 mg, or 800 mg, and the substance Z is administered to the subject at a fixed dose (referring to a single dose) of 300 mg, 400 mg, 500 mg, 600 mg, 800 mg, 900 mg, 1000 mg, 1100 mg, or 1200 mg.

In some embodiments, the substance Y is pretomanid or a pharmaceutically acceptable salt thereof, and the

substance Z is linezolid or a pharmaceutically acceptable salt thereof; the substance X is administered to the subject at a fixed dose (referring to a single dose) of 450 mg, the substance Y is administered to the subject at a fixed dose (referring to a single dose) of 100 mg, 200 mg, 300 mg, 400 mg, 500 mg, 600 mg, or 800 mg, and the substance Z is administered to the subject at a fixed dose (referring to a single dose) of 300 mg, 400 mg, 500 mg, 600 mg, 800 mg, 900 mg, 1000 mg, 1100 mg, or 1200 mg.

In some embodiments, the substance Y is delamanid or a pharmaceutically acceptable salt thereof, and the substance Z is linezolid or a pharmaceutically acceptable salt thereof; the substance X is administered to the subject at a fixed dose (referring to a single dose) of 150 mg, the substance Y is administered to the subject at a fixed dose (referring to a single dose) of 100 mg, 200 mg, 300 mg, 400 mg, 500 mg, 600 mg, or 800 mg, and the substance Z is administered to the subject at a fixed dose (referring to a single dose) of 300 mg, 400 mg, 500 mg, 600 mg, 800 mg, 900 mg, 1000 mg, 1100 mg, or 1200 mg.

In some embodiments, the substance Y is delamanid or a pharmaceutically acceptable salt thereof, and the substance Z is linezolid or a pharmaceutically acceptable salt thereof; the substance X is administered to the subject at a fixed dose (referring to a single dose) of 300 mg, the substance Y is administered to the subject at a fixed dose (referring to a single dose) of 100 mg, 200 mg, 300 mg, 400 mg, 500 mg, 600 mg, or 800 mg, and the substance Z is administered to the subject at a fixed dose (referring to a single dose) of 300 mg, 400 mg, 500 mg, 600 mg, 800 mg, 900 mg, 1000 mg, 1100 mg, or 1200 mg.

In some embodiments, the substance Y is delamanid or a pharmaceutically acceptable salt thereof, and the substance Z is linezolid or a pharmaceutically acceptable salt thereof; the substance X is administered to the subject at a fixed dose (referring to a single dose) of 450 mg, the substance Y is administered to the subject at a fixed dose (referring to a single dose) of 100 mg, 200 mg, 300 mg, 400 mg, 500 mg, 600 mg, or 800 mg, and the substance Z is administered to the subject at a fixed dose (referring to a single dose) of 300 mg, 400 mg, 500 mg, 600 mg, 800 mg, 900 mg, 1000 mg, 1100 mg, or 1200 mg.

In some embodiments, the aforementioned fixed dose of the substance X may be administered at a frequency of once a day, every other day, three times a week, twice a week, or once a week.

In some embodiments, the substance X is administered at a frequency of once a day.

In some embodiments, the substance X is administered orally according to the aforementioned fixed dose and frequency.

In some embodiments, the aforementioned fixed dose of the substance Y may be administered at a frequency of once a day, every other day, three times a week, twice a week, or once a week.

In some embodiments, the substance Y is administered at a frequency of once a day.

In some embodiments, the substance Y is administered orally according to the aforementioned fixed dose and frequency.

In some embodiments, the aforementioned fixed dose of the substance Z may be administered at a frequency of once a day, every other day, three times a week, twice a week, or once a week.

In some embodiments, the substance Z is administered at a frequency of once a day.

In some embodiments, the substance Z is administered orally according to the aforementioned fixed dose and frequency.

## Definitions and Explanations

Unless otherwise stated, the following terms and phrases used herein are intended to have the following meanings. A specific term or phrase should not be considered uncertain or unclear in the absence of a specific definition, but rather should be understood according to its ordinary meaning. When a trade name appears herein, it is intended to refer to its corresponding commodity or the active ingredient thereof.

The term "optional" or "optionally" as used herein means that the subsequently described event or circumstance may or may not occur.

The term "therapeutically effective amount" as used herein refers to an amount of a compound that, when administered to a patient, is sufficient to effectively prevent or treat the diseases or conditions described herein. The amount of a compound constituting a "therapeutically effective amount" will vary depending on the compound, the condition and its severity, and the age of the patient to be treated, but can be adjusted as needed by those skilled in the art.

The term "pharmaceutical composition" as used herein refers to a composition containing specified active ingredients that can be prepared into the same dosage form.

The term "subject in need thereof" as used herein refers to any animal, preferably a mammal, and most preferably a human, that will receive or has received administration of the compound or composition according to the embodiments of the present disclosure. As used herein, the term "mammal" includes any mammal. Examples of mammals include, but are not limited to, cattle, horses, sheep, pigs, cats, dogs, mice, rats, rabbits, guinea pigs, monkeys, humans, and the like, with humans being most preferred.

The term "pharmaceutically acceptable carrier or medium" as used herein refers to excipients and additives used in the production of pharmaceuticals and the formulation of prescriptions, being all substances included in a pharmaceutical preparation other than the active ingredients. Reference may be made to the Pharmacopoeia of the People's Republic of China (2020 Edition), Volume IV, or the Handbook of Pharmaceutical Excipients (Raymond C Rowe, 2009 Sixth Edition).

The term "pharmaceutically acceptable" as used herein means that the acids or bases (used for preparing salts), solvents, auxiliary materials, and the like are generally non-toxic, safe, and suitable for use by a patient. The "patient" described is preferably a mammal, and more preferably a human.

The term "pharmaceutically acceptable salt" as used herein refers to a salt prepared from a compound with a relatively non-toxic, pharmaceutically acceptable acid or base. When a compound contains a relatively acidic functional group, a base addition salt can be obtained by contacting the neutral form of such a compound with a sufficient amount of a pharmaceutically acceptable base in a pure solution or a suitable inert solvent. Pharmaceutically acceptable base addition salts include, but are not limited to: lithium salts, sodium salts, potassium salts, calcium salts, aluminum salts, magnesium salts, zinc salts, bismuth salts, ammonium salts, and diethanolamine salts. When a compound contains a relatively basic functional group, an acid addition salt can be obtained by contacting the neutral form of such a compound with a sufficient amount of a pharmaceutically acceptable acid in a pure solution or a suitable inert solvent. The pharmaceutically acceptable acids include inorganic acids, and said inorganic acids include, but are not limited to: hydrochloric acid, hydrobromic acid, hydroiodic acid, nitric acid, carbonic acid, bicarbonate, phosphoric acid, monohydrogen phosphate, dihydrogen phosphate, phosphorous acid, sulfuric acid, bisulfate, and the like. The pharmaceutically acceptable acids include organic acids, and said organic acids include, but are not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acid citrate, oleic acid, tannic acid, pantothenic acid, bitartrate, ascorbic acid, gentisic acid, gluconic acid, saccharic acid, formic acid, ethanesulfonic acid, pamoic acid (i.e., 4,4'-methylene-bis(3-hydroxy-2-naphthoic acid)), amino acids (such as glutamate, arginine), and the like. When a compound contains both relatively acidic and relatively basic functional groups, it can be converted into base addition salts or acid addition salts. Specific reference can be made to Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The definitions of the terms "compound I" and "pharmaceutically acceptable salt of compound I" as used herein include all forms of the compound of formula (I) or salts thereof, including crystalline and amorphous forms, isomorphs, polymorphs, solvates, and hydrates of the compound of formula (I), as well as crystalline and amorphous forms, isomorphs, polymorphs, solvates, and hydrates of the pharmaceutically acceptable salt of compound I.

The term "solvate" as used herein describes a non-covalent or readily reversible combination or dispersion form or dispersed phase between a solvent and a solute. It is understood that a solvate may be in the form of a solid, a slurry (such as a suspension or dispersion), or a solution. Non-limiting examples of solvents include ethanol, methanol, propanol, acetonitrile, dimethyl ether, diethyl ether, tetrahydrofuran, dichloromethane, and water. When the solvent is water, the term "hydrate" is used.

The term "administered simultaneously" as used herein refers to the simultaneous administration of each substance in a pharmaceutical combination contained in a single pharmaceutical composition, or the simultaneous administration of individual pharmaceutical compositions each comprising one substance of a pharmaceutical combination. For example, in the case of a pharmaceutical combination comprising substance X and substance Y, simultaneous administration means that substance X and substance Y are contained in a single pharmaceutical composition and administered simultaneously; or a "separate pharmaceutical composition comprising substance X" and a "separate pharmaceutical composition comprising substance Y" are administered simultaneously. As another example, in the case of a pharmaceutical composition comprising substance X, substance Y, and substance Z, simultaneous administration means that substance X, substance Y, and substance Z are contained in a single pharmaceutical composition and administered simultaneously; or a "separate pharmaceutical composition comprising substance X," a "separate pharmaceutical composition comprising substance Y," and a "separate pharmaceutical composition comprising substance Z" are administered simultaneously.

The term "administered separately" as used herein refers to the separate administration of individual pharmaceutical compositions each comprising one substance of a pharmaceutical combination at different times, wherein said separate administration may be close in time or distant in time. For example, in the case of a pharmaceutical combination comprising substance X and substance Y, separate administration means that a "separate pharmaceutical composition comprising substance X" and a "separate pharmaceutical composition comprising substance Y" are administered separately at different times. As another example, in the case of a pharmaceutical composition comprising substance X, substance Y, and substance Z, separate administration means that a "separate pharmaceutical composition comprising substance X," a "separate pharmaceutical composition comprising substance Y," and a "separate pharmaceutical composition comprising substance Z" are administered separately at different times.

Regardless of whether it is simultaneous administration or separate administration, the administration regimen (including administration route, administration dose, administration interval, etc.) of each substance in the pharmaceutical combination may be the same or different, and can be adjusted by those skilled in the art as needed to provide the optimal therapeutic effect.

The "certain mass" in the term "a certain mass (e.g., 150 mg) of a compound or a pharmaceutically acceptable salt thereof" as used herein is calculated based on the free base of the active ingredient in the compound or the pharmaceutically acceptable salt thereof. For example, the statement that "the pharmaceutical combination comprises 150 mg of the compound of formula (I) or a pharmaceutically acceptable salt thereof" means that the mass of the free base (i.e., the compound of formula (I)) of the active ingredient in the compound of formula (I) or the pharmaceutically acceptable salt thereof comprised in the pharmaceutical combination is 150 mg.

The term "fixed dose" as used herein is calculated based on the free base of the active ingredient in the substance administered. For example, the statement that "substance Y is administered to the subject at a fixed dose (referring to a single dose) of 100 mg" means that the mass of the free base of the active ingredient in the substance Y administered to the subject is 100 mg.

The term "pyrazinamide" as used herein has the chemical formula C5H5N3O and the CAS Registry Number 98-96-4.

The term "moxifloxacin" as used herein has the chemical formula C21H24FN3O4 and the CAS Registry Number 151096-09-2.

The term "linezolid" as used herein has the chemical formula C16H20FN3O4 and the CAS Registry Number 165800-03-3.

The term "Pretomanid" as used herein has the chemical formula C14H12F3N3O5 and the CAS Registry Number 187235-37-6.

The term "delamanid" as used herein has the chemical formula C25H25F3N4O6 and the CAS Registry Number 681492-22-8.

Without departing from common knowledge in the art, the aforementioned preferred conditions can be combined arbitrarily to obtain various preferred embodiments of the present disclosure.

The positive and progressive effects of the present disclosure are that it provides a novel pharmaceutical combination comprising compound I and other anti-tuberculosis drugs, which is conducive to overcoming drug resistance problems, improving efficacy, and shortening treatment time; it is also beneficial for improving patient compliance, reducing side effects, lowering costs, and increasing cure rates. Compared with most single-agent treatments, the pharmaceutical combination of the present invention exhibits a significant improvement in *in vivo* efficacy, and certain pharmaceutical combinations show particularly remarkable *in vivo* efficacy.

DETAILED DESCRIPTION

The present disclosure is further illustrated below by way of examples, but the present disclosure is not thereby limited to the scope of the described examples. Experimental methods in the following examples for which specific conditions are not noted are selected according to conventional methods and conditions, or according to commercial instructions.

The following abbreviations are used in the following examples and, unless otherwise specified, have the indicated definitions: CFU is colony-forming unit; INH is isoniazid; RFP is rifampicin; PZA is pyrazinamide; MFX is moxifloxacin; LZD is linezolid; BDQ is bedaquiline; PMD is pretomanid; DLM is delamanid.

Materials and methods

1) Strains: *Mycobacterium tuberculosis* standard strain $H_{37}Rv$ (ATCC 27294) was purchased, and then deposited in the Pharmacology Laboratory of the Beijing Tuberculosis and Thoracic Tumor Research Institute. The clinical isolates of *Mycobacterium tuberculosis* were provided by the National Tuberculosis Clinical Laboratory of the Beijing Tuberculosis and Thoracic Tumor Research Institute. Basic information for each clinical strain is shown in Table 1.

Table 1: Basic information of *Mycobacterium tuberculosis* clinical isolates

| Strain No. | Medical record No. | Region | Collection time | Strain No. | Medical record No. | Region | Collection time |
|---|---|---|---|---|---|---|---|
| 30031 (clinical sensitive strain) | 144602 | Hebei | 2019.4 | 29925 (clinical drug-resistant strain) | 144369 | Shaanxi | 2019.4 |
| 30091 (clinical sensitive strain) | 144820 | Anhui | 2019.4 | 30611 (clinical drug-resistant strain) | 143360 | Beijing | 2019.2 |

2) Reagents

Alamar Blue (AbD Serotec, UK);

Middlebrook OADC broth, 7H9 liquid culture medium (Becton Dickinson Company, USA);

Middlebrook OADC broth, 7H10 solid culture medium (Becton Dickinson Company, USA);

DMSO (Beijing Solebo Technology Co., Ltd.);

Glycerol (Beijing Chemical Factory);

Tween-80 (Beijing Solarbio Science & Technology Co., Ltd.).

3) Compounds

INH, RFP (Sigma, USA); Compound I (Shanghai Jiatan Pharmaceutical, or obtainable by the preparation method disclosed in WO2017121323A1); BDQ, MFX, PMD (Shanghai Biochempartner); DLM (TargetMol, USA); LZD (ArRPharm).

4) Preparation of drug stock solutions

A suitable amount of anti-tuberculosis drug powder as shown in the table below was weighed into a 15 mL sterile centrifuge tube. Water-soluble drugs were dissolved by adding ultrapure water, and fat-soluble drugs were dissolved by adding DMSO. After ultrasonication and membrane filtration, the stock solutions of each anti-tuberculosis drug were aliquoted into sterile EP tubes and stored in a -80°C freezer for later use. The specific preparation methods for each drug stock solution are shown in Table 2.

Table 2: Preparation method for each drug stock solution

| Name | Solvent | Stock solution | Name | Solvent | Stock solution |
|---|---|---|---|---|---|
| INH | Autoclaved | 1.0 | LZD | DMSO | 1.0 |
| RFP | DMSO | 1.0 | DLM | DMSO | 1.0 |
| Compound | DMSO | 1.0 | MFX | DMSO | 1.0 |
| BDQ | DMSO | 1.0 | PMD | DMSO | 1.0 |

**Example 1: Determination of MIC of anti-tuberculosis drugs**

1) Culture and subculture of *Mycobacterium tuberculosis*

Frozen stock solutions of the standard strain $H_{37}Rv$ and four clinical strains (Strain Nos.: 30031, 30091, 29925,

30611) were removed from a -80°C freezer and allowed to thaw at room temperature. After complete thawing, 200 μL of each bacterial suspension was aspirated and added to 7H9 liquid medium containing 0.05% Tween-80. The cultures were incubated in a constant temperature incubator at 37°C with 5% $CO_2$ for approximately 2 weeks until reaching the logarithmic growth phase.

2) Serial dilution of drug stock solutions

Due to the extremely low MIC value of DLM, DLM was diluted 256-fold in advance. First, 100 μL of 7H9 liquid medium was added to the first 6 wells of Row A of a 96-well plate, and 200 μL of 7H9 liquid medium was added to the remaining 6 wells (the first 6 wells would subsequently receive 100 μL of the diluted bacterial suspension to serve as positive control wells, while the remaining 6 wells served as negative control wells). Next, 198 μL of 7H9 liquid medium was added to the microwells in the first column of Rows B-H of the 96-well plate, and 100 μL of 7H9 liquid medium was added to all other microwells. 2 μL of each drug stock solution was added to the microwells in the first column of Rows B-H. After the drug solution and the 7H9 liquid medium were thoroughly mixed, 100 μL of the mixed solution was aspirated and transferred to the next column using a two-fold serial dilution method. This operation was repeated until the last column of microwells, and the final 100 μL of liquid was discarded.

3) Determination and dilution of bacterial suspension concentration

One standard strain and four clinical strains cultured to the logarithmic growth phase were removed from the constant temperature incubator at 37°C with 5% $CO_2$. Within a biosafety cabinet, 200 μL of each bacterial suspension and 7H9 liquid medium were aspirated into a 96-well plate, with 2 duplicate wells set for each sample. The 96-well plate was placed in a multi-mode microplate reader to determine the OD value of each well at a wavelength of 570 nm, where OD(strain) = OD(bacterial suspension) - OD(7H9 medium). The final concentration of each strain was set to $1\times10^5$ CFU/mL. Corresponding volumes of 7H9 liquid medium were added to dilute each bacterial suspension, where OD = 0.1 is equivalent to $1\times10^7$ CFU/mL.

Except for the negative control wells, the diluted bacterial suspension was added to all other wells at a volume of 100 μL/well. At this point, the total volume of all wells was 200 μL. The plates were sealed with sealing film and cultured in a constant temperature incubator at 37°C with 5% $CO_2$ for 7 days.

4) Addition of Alamar Blue indicator

The 96-well plate was removed from the incubator, and the entire process was performed protected from light. Alamar Blue indicator was added at a volume of 20 μL/well, and 20% Tween-80 was added at a volume of 12.5 μL/well. After sealing, the 96-well plate was placed in a constant temperature incubator at 37°C with 5% $CO_2$ for incubation. Color changes were observed the following day.

5) Determination of fluorescence values

The 96-well plate was placed in a multi-mode microplate reader, and the fluorescence value of each microwell was measured at an excitation wavelength of 530 nm and an emission wavelength of 590 nm, while the color of each well was recorded. Blue wells represent no growth, and pink wells represent growth. Purplish-red wells were further incubated at 37°C for 24 hours; if they did not turn pink and their adjacent blue wells remained blue, they were recorded as having growth.

The MIC results of the anti-tuberculosis drugs (Compound I, BDQ, INH, RFP, LZD, DLM, MFX, and PMD) against the tuberculosis strains determined by the MABA method are shown in Table 3.

Table 3: *In vitro* minimal inhibitory concentrations (μg/mL) of anti-tuberculosis drugs against H37Rv and 4 clinical strains

| Strain | Compound I | BDQ | INH | RFP | LZD | DLM | MFX | PMD |
|---|---|---|---|---|---|---|---|---|
| $H_{37}Rv$ | 0.15 | 0.04 | 0.04 | 0.04 | 0.29 | 0.002 | 0.15 | 0.08 |
| 30031 | 0.08 | 0.03 | 0.08 | 0.08 | 0.45 | 0.005 | 0.08 | 0.23 |
| 30091 | 0.07 | 0.02 | 0.02 | 0.04 | 0.15 | 0.01 | 0.07 | 0.3 |
| 29925 | 0.16 | 0.04 | >20 | >20 | 0.39 | 0.03 | 4.78 | 0.12 |
| 30611 | 0.07 | 0.02 | >20 | >20 | 0.14 | 0.01 | 0.07 | 0.26 |

**Example 2: Determination of FICI for two-drug combinations comprising compound I by Two-Dimensional Checkerboard Method**

1) Checkerboard design

Based on the MIC values of each drug when used alone, a total of 7 concentration gradients were set, with a concentration range of 2 × MIC to 1/32 × MIC. The drugs were distributed in a checkerboard lattice as shown in Table 4. In a 96-well plate, drug A (LZD, DLM, MFX, PMD) at 2 × MIC to 1/32 × MIC was added to wells A3-H9, and drug B (compound I) at 2 × MIC to 1/32 × MIC was added to wells B2-H9. Drug A was two-fold diluted horizontally, and drug B was two-fold diluted vertically. Columns B10-H10 and B11-H11 served as negative control wells and positive control wells, respectively.

Table 4: Two-dimensional checkerboard design

| No. | 2 | 3 | 4 | 5 | 6 | 7 | 8 | 9 |
|---|---|---|---|---|---|---|---|---|
| A | | X1 | X2 | X3 | X4 | X5 | X6 | X7 |
| B | Y1 | X1+Y1 | X2+Y1 | X3+Y1 | X4+Y1 | X5+Y1 | X6+Y1 | X7+Y1 |
| C | Y2 | X1+Y2 | X2+Y2 | X3+Y2 | X4+Y2 | X5+Y2 | X6+Y2 | X7+Y2 |
| D | Y3 | X1+Y3 | X2+Y3 | X3+Y3 | X4+Y3 | X5+Y3 | X6+Y3 | X7+Y3 |
| E | Y4 | X1+Y4 | X2+Y4 | X3+Y4 | X4+Y4 | X5+Y4 | X6+Y4 | X7+Y4 |
| F | Y5 | X1+Y5 | X2+Y5 | X3+Y5 | X4+Y5 | X5+Y5 | X6+Y5 | X7+Y5 |
| G | Y6 | X1+Y6 | X2+Y6 | X3+Y6 | X4+Y6 | X5+Y6 | X6+Y6 | X7+Y6 |
| H | Y7 | X1+Y7 | X2+Y7 | X3+Y7 | X4+Y7 | X5+Y7 | X6+Y7 | X7+Y7 |

2) Plating of drugs to be tested

Plating of drug A: All drug stock solutions were diluted to 10 × MIC in advance. 95 μL of 7H9 liquid medium was added to wells B2-B9 of a 96-well plate, and 50 μL of 7H9 liquid medium was added to wells C2-H9. 5 μL of the diluted drug A was then added to wells B2-B9, and the drug solution and 7H9 liquid medium were thoroughly mixed. 50 μL of the mixed solution was transferred to the next row using a two-fold dilution method; this operation was repeated until the microwells of row H, and the final 50 μL of liquid was discarded.

Plating of drug B: All drug stock solutions were diluted to 10 × MIC in advance. 125 μL of 7H9 liquid medium was added to wells A3-H3 of the 96-well plate, and 65 μL of 7H9 liquid medium was added to wells A4-H9. 5 μL of the diluted drug A was then added to wells A3-H3, and the drug solution and 7H9 liquid medium were thoroughly mixed. 65 μL of the mixed solution was transferred to the next column using a two-fold dilution method; this operation was repeated until the microwells of column 9, and the final 65 μL of liquid was discarded.

3) Crossover of drug A and drug B

After drug A and drug B were plated in the manner described above, 50 μL of diluted drug B was pipetted and added to the 96-well plate containing drug A according to the corresponding wells. 50 μL of 7H9 liquid medium was supplemented to the single-drug wells B2-H2 and A3-A9. At this point, the liquid volume in each well of the 96-well plate was 100 μL. 200 μL of 7H9 liquid medium was added to the microwells in column 10 as negative control wells, and 100 μL of 7H9 liquid medium was added to the microwells in column 11 (to which 100 μL of diluted bacterial suspension would subsequently be added) as positive control wells.

4) Determination of bacterial suspension concentration and addition

Refer to Example 1 for specific procedural steps.

5) Addition of fluorescent indicator

Refer to Example 1 for specific procedural steps.

6) Determination of drug interactions

24-Hours after adding the Alamar Blue indicator, the 96-well plate was removed from the incubator. First, the color changes of drug A alone and drug B alone were observed to determine their respective MIC wells. The effect of the two-drug combination was determined by calculating the FICI value according to the calculation formula:

$$FICI = \frac{MICA\ combined}{MICA\ alone} + \frac{MICB\ combined}{MICB\ alone}$$

Where $MIC_{A\ alone}$ and $MIC_{B\ alone}$ represent the MIC of drug A and drug B against *Mycobacterium tuberculosis* when used alone, respectively; $MIC_{A\ combined}$ and $MIC_{B\ combined}$ represent the lowest concentrations of drug A and drug B that prevent the color from changing from blue to pink after drug A and drug B are used in combination. The criteria for assessing the *in vitro* activity of the two-drug combination are as follows: FICI ≤ 0.5 indicates synergy; 0.5 < FICI < 1 indicates partial synergy; FICI = 1 indicates an additive effect; 1 < FICI < 4 indicates no interaction; FICI ≥ 4 indicates antagonism. Partial results of the FICI of two-drug combinations comprising compound I determined by the checkerboard method are shown in Table 5 below.

Table 5: FICI results of two-drug combinations comprising compound I as determined by the checkerboard method

| Strain | Drug B | MIC (μg/mL) | | *In vitro* activity with compound I | |
|---|---|---|---|---|---|
| | | $MIC_{Compound\ I\ combined}$ /$MIC_{Compound\ I\ alone}$ | $MIC_{B\ combined}$ /$MIC_{B\ alone}$ | FICI | Result |
| $H_{37}Rv$ | DLM | 0.25 | 0.5 | 0.75 | Partial synergy |
| 30031 | PMD | 0.25 | 0.5 | 0.75 | Partial synergy |
| 30031 | DLM | 0.25 | 0.25 | 0.5 | Synergy |
| 30091 | PMD | 0.125 | 0.5 | 0.625 | Partial synergy |
| 30091 | DLM | 0.25 | 0.25 | 0.5 | Synergy |
| 29925 | DLM | 0.25 | 0.5 | 0.75 | Partial synergy |

The test results indicate that compound I, in combination with other anti-tuberculosis drugs, can synergistically inhibit the growth of *Mycobacterium tuberculosis.* In particular, the synergistic inhibitory effect on the growth of *Mycobacterium tuberculosis* is more significant when compound I is used in combination with DLM and PMD.

**Example 3: Determination of FICI for three-drug combinations comprising compound I by Three-Dimensional Checkerboard Method**

1) Checkerboard design

Based on the MIC values of each drug when used alone, a total of 6 concentration gradients were set, with a concentration range of 2×MIC to 1/16×MIC. The drugs were distributed in a checkerboard lattice as shown in Table 6. In a 96-well plate, drug A (DLM, PMD) at 2×MIC to 1/16×MIC was added to wells A3-G8, drug B (compound I) at 2×MIC to 1/16×MIC was added to wells B2-G8, and drug C (LZD) at 2×MIC to 1/16×MIC was added to wells H3-H8. Drug A was two-fold diluted horizontally, and drug B was two-fold diluted vertically. The concentration of drug C remained constant in the combination wells. Columns B9-G9 and B10-G10 served as negative control wells and positive control wells, respectively.

Table 6: Three-dimensional checkerboard design

| No . | 2 | 3 | 4 | 5 | 6 | 7 | 8 |
|---|---|---|---|---|---|---|---|
| A | | X1 | X2 | X3 | X4 | X5 | X6 |
| B | Y 1 | X1+Y1+Z 4 | X2+Y1+Z 4 | X3+Y1+Z 4 | X4+Y1+Z 4 | X5+Y1+Z 4 | X6+Y1+Z 4 |
| C | Y 2 | X1+Y2+Z 4 | X2+Y2+Z 4 | X3+Y2+Z 4 | X4+Y2+Z 4 | X5+Y2+Z 4 | X6+Y2+Z 4 |
| D | Y 3 | X1+Y3+Z 4 | X2+Y3+Z 4 | X3+Y3+Z 4 | X4+Y3+Z 4 | X5+Y3+Z 4 | X6+Y3+Z 4 |

(continued)

| No . | 2 | 3 | 4 | 5 | 6 | 7 | 8 |
|---|---|---|---|---|---|---|---|
| E | Y 4 | X1+Y4+Z 4 | X2+Y4+Z 4 | X3+Y4+Z 4 | X4+Y4+Z 4 | X5+Y4+Z 4 | X6+Y4+Z 4 |
| F | Y 5 | X1+Y5+Z 4 | X2+Y5+Z 4 | X3+Y5+Z 4 | X4+Y5+Z 4 | X5+Y5+Z 4 | X6+Y5+Z 4 |
| G | Y 6 | X1+Y6+Z 4 | X2+Y6+Z 4 | X3+Y6+Z 4 | X4+Y6+Z 4 | X5+Y6+Z 4 | X6+Y6+Z 4 |
| H | | Z1 | Z2 | Z3 | Z4 | Z5 | Z6 |

2) Plating of drugs to be tested

Plating of drug A: All drug stock solutions were diluted to 10 × MIC in advance. 78 μL of 7H9 liquid medium was added to wells B2-B8 of a 96-well plate, and 40 μL of 7H9 liquid medium was added to wells C2-G8. 2 μL of the diluted drug A was then added to wells B2-B8, and the drug solution and 7H9 liquid medium were thoroughly mixed. 40 μL of the mixed solution was transferred to the next row using a two-fold dilution method; this operation was repeated until the microwells of row G, and the final 40 μL of liquid was discarded.

Plating of drug B: All drug stock solutions were diluted to 10 × MIC in advance. 118 μL of 7H9 liquid medium was added to wells A3-G3 of the 96-well plate, and 60 μL of 7H9 liquid medium was added to wells A4-G8. 2 μL of the diluted drug A was then added to wells A3-G3, and the drug solution and 7H9 liquid medium were thoroughly mixed. 60 μL of the mixed solution was transferred to the next column using a two-fold dilution method; this operation was repeated until the microwells of column 8, and the final 60 μL of liquid was discarded.

3) Crossover of drug A and drug B

After drug A and drug B were plated in the manner described above, 40 μL of diluted drug B was pipetted and added to the 96-well plate containing drug A according to the corresponding wells. 60 μL of 7H9 liquid medium was supplemented to the single-drug wells B2-G2 and A3-A8. At this point, the liquid volume in each well of the 96-well plate was 100 μL. 200 μL of 7H9 liquid medium was added to the microwells in column 9 as negative control wells, and 100 μL of 7H9 liquid medium was added to the microwells in column 10 (to which 100 μL of diluted bacterial suspension would subsequently be added) as positive control wells.

4) Addition and dilution of drug C

Single-drug wells for drug C: The stock solution of drug C was diluted to 4×MIC in advance. 200 μL was pipetted into well H3, and 100 μL of 7H9 liquid medium was added to wells H4-H8. Drug C was then two-fold diluted, and 100 μL of the mixed drug solution was pipetted from well H8 and discarded.

Combination wells for drug C: The first well of the single-drug wells for drug C was first diluted 16-fold, and then the diluted drug C was added to wells B3-G8 at a volume of 20 μL/well. At this point, the concentration of drug C in the combination wells was 1/4×MIC.

5) Determination of bacterial suspension concentration and addition

Refer to Example 1 for specific procedural steps.

6) Addition of Alamar Blue indicator

The addition of the chromogenic agent was performed with reference to Example 1.

7) Criteria for determining drug interactions

24-Hours after adding the Alamar Blue indicator, the 96-well plate was removed from the incubator. First, the color changes in the single-drug wells of drug A, drug B, and drug C were observed to determine their respective MIC wells. The effect of the three-drug combination was determined by calculating the FICI value according to the calculation formula:

$$\mathrm{FICI} = \frac{\text{MICA combined}}{\text{MICA alone}} + \frac{\text{MICB combined}}{\text{MICB alone}} + \frac{\text{MICC combined}}{\text{MICC alone}}$$

where $MIC_{A\ alone}$, $MIC_{B\ alone}$, and $MICc_{alone}$ represent the MIC of drug A, drug B, and drug C against each strain when used alone, respectively; $MIC_{A\ combined}$ and $MIC_{B\ combined}$ represent the lowest concentrations of drug A and drug B that prevent the color from changing from blue to pink after drug A and drug B are used in combination. When the MIC value of drug C appeared in well H4, the drug concentration of $MIC_{C\ combined}$ was 1/4×MIC of drug C; if it appeared in well H5, the drug concentration of $MICc_{combined}$ was 1/8×MIC of drug C. The criteria for assessing the *in vitro* activity of the three-drug combination are as follows: FICI ≤ 0.75 indicates synergy; 0.75 < FICI < 4 indicates an additive effect/no interaction; FICI ≥ 4 indicates antagonism. Partial results of the FICI of three-drug combinations comprising compound I determined by the checkerboard method are shown in Table 7 below.

Table 7: FICI results of three-drug combinations comprising compound I as determined by the checkerboard method

| Strain | Combination | $MIC_{alone}$ | $MIC_{combined}$ | FICI | Result |
|---|---|---|---|---|---|
| 30031 | Compound I/PMD/LZD | 0.08/0.23/0.4 5 | 0.02/0.06/0.11 | 0.75 | Synergy |
| 30031 | Compound I/DLM/LZD | 0.08/0.005/0. 45 | 0.02/0.001/0.11 | 0.75 | Synergy |
| 30091 | Compound I/DLM/LZD | 0.07/0.0110.1 5 | 0.02/0.003/0.04 | 0.75 | Synergy |

The test results indicate that the combination of compound I, PMD, and LZD, or the combination of compound I, DLM, and LZD, can synergistically inhibit the growth of *Mycobacterium tuberculosis.*

**Example 4: *In vivo* activity of two-drug combinations**

SPF-grade female BALB/c mice (6-8 weeks old, body weight 19.52 ± 0.52 g) were infected with the *Mycobacterium tuberculosis* standard strain $H_{37}Rv$ (ATCC 27294) via aerosol inhalation using an aerosol infection apparatus (Model: Glas-Collin halation Exposure System 099C A4224) at an infection dose of $2\times10^7$ CFU/mL. On the 15th day and at the 4th week post-infection, 6 mice were euthanized respectively to determine the bacterial counts at the corresponding time points.

Treatment began 4 weeks after aerosol infection. The mice were divided into groups according to different treatment regimens, with 12 mice per group: untreated group (0.5% sodium carboxymethyl cellulose solution), compound I group (20 mg/kg), LZD group (100 mg/kg), MFX group (50 mg/kg), PMD group (100 mg/kg), DLM group (5 mg/kg), PZA group (150 mg/kg), compound I (20 mg/kg) combined with LZD (100 mg/kg) group, compound I (20 mg/kg) combined with MFX (50 mg/kg) group, compound I (20 mg/kg) combined with PMD (100 mg/kg) group, compound I (20 mg/kg) combined with DLM (5 mg/kg) group, and compound I (20 mg/kg) combined with PZA (150 mg/kg) group. All drugs were dissolved or suspended in 0.5% sodium carboxymethyl cellulose solution. Dosages for five administrations per week were prepared at one time and stored at 4°C. Oral gavage administration was performed daily from Monday to Friday with an administration volume of 0.2 mL. Six mice from each group were euthanized after 4 and 8 weeks of treatment, and CFU counts of *Mycobacterium tuberculosis* in the whole lungs and spleens were performed to evaluate the bactericidal activity of each regimen. The specific results for the lungs are shown in Table 8:

Table 8: CFU counts in lungs of BALB/c mice at various time points (x ± s) (Values are mean±SD, standard deviation)

| Group | Average log10 CFU count | | | |
|---|---|---|---|---|
| | D-15 | D0 | 4 weeks | 8 weeks |
| Untreated group | 4.95±0.18 | 5.14±0.19 | 6.05±0.33 | 6.18±0.19 |
| Compound I | | | 4.63±0.27 | 1.44±0.49 |
| LZD | | | 5.74±0.17 | 5.00±0.24 |
| MFX | | | 5.18±0.22 | 4.79±0.16 |
| PMD | | | 5.49±0.64 | 3.89±0.09 |
| DLM | | | 5.88±0.36 | 5.79±0.19 |
| PZA | | | 4.07±0.22 | 3.30±0.29 |
| Compound I+LZD | | | 5.05±0.10 | 3.54±0.19 |
| Compound I+MFX | | | 4.51±0.40 | 1.69±0.17 |
| Compound I+PMD | | | 4.09±0.25 | 1.40±0.36 |

(continued)

| Group | Average log10 CFU count | | | |
|---|---|---|---|---|
| | D-15 | D0 | 4 weeks | 8 weeks |
| Compound I+DLM | | | 4.95±0.16 | 2.72±0.34 |
| Compound I+PZA | | | 2.46±0.64 | Negative |
| D-15: 15 days post-infection; D0: date of administration and treatment; 4 weeks/8 weeks: 4 weeks/8 weeks post-administration. | | | | |

According to the above test results, all five combination regimens comprising compound I showed good anti-tuberculosis activity in mice. Combination with compound I significantly enhanced the antibacterial activities of LZD, DLM, PZA, and PMD. After 8 weeks of treatment, the therapeutic effects of the combination regimens were superior to those of the corresponding single-drug regimens of LZD, DLM, PZA, and PMD. In particular, the treatment group of compound I combined with PZA achieved pulmonary sterilization after 8 weeks of oral administration, and lung cultures from all mice in this group tested negative.

**Example 5: *In vivo* activity of three-drug combinations**

SPF-grade female BALB/c mice (6-8 weeks old, body weight 20.73 ± 0.47 g) were infected with the *Mycobacterium tuberculosis* standard strain $H_{37}Rv$ (ATCC 27294) via aerosol inhalation using an aerosol infection apparatus (Model: Glas-Collin halation Exposure System 099C A4224) at an infection dose of $5.15\times10^7$ CFU/mL. On the 14th day and at the 4th week post-infection, 3 mice and 5 mice were euthanized respectively to determine the bacterial counts at the corresponding time points.

Treatment began 4 weeks after aerosol infection. The mice were divided into groups according to different treatment regimens, with 15 mice per group: untreated group (0.5% sodium carboxymethyl cellulose solution), HRZ group (25 mg/kg INH + 50 mg/kg RFP + 150 mg/kg PZA), DLM group (5 mg/kg), compound I group (25 mg/kg), compound I (25 mg/kg) combined with DLM (5 mg/kg) group, compound I (25 mg/kg) combined with DLM (5 mg/kg) and LZD (100 mg/kg) group, BDQ (25 mg/kg) combined with DLM (5 mg/kg) and LZD (100 mg/kg) group, BDQ (25 mg/kg) combined with PMD (100 mg/kg) and LZD (100 mg/kg) group, and compound I (25 mg/kg) combined with PMD (100 mg/kg) and LZD (100 mg/kg) group. All drugs were dissolved or suspended in 0.5% sodium carboxymethyl cellulose solution. Dosages for five administrations per week were prepared at one time and stored at 4°C. Oral gavage administration was performed daily from Monday to Friday with an administration volume of 0.2 mL.

Five mice from each group were euthanized after 4 weeks, 8 weeks, and 12 weeks of treatment, and CFU counts in the lungs and spleens were performed to evaluate the bactericidal activity of each regimen. The specific results are shown in Table 9 and Table 10:

Table 9: CFU counts in lungs of BALB/c mice at various time points (Values are mean±SD, standard deviation)

| Group | Average log10 CFU count | | | | |
|---|---|---|---|---|---|
| | D-16 | D0 | 4 weeks | 8 weeks | 12 weeks |
| Untreated group | 5.15±0.12 | 5.25±0.33 | 7.64±0.11 | 7.35±0.41 | 8.25±0.52 |
| HRZ | | | 2.37±0.36 | Negative | 1.51±0.00 |
| DLM | | | 6.33±0.16 | 5.74±0.19 | 5.71±0.19 |
| Compound I | | | 4.55±0.19 | 2.12±0.40 | - |
| Compound I+DLM | | | 4.65±0.17 | 2.80±0.14 | 0.80±0.14 |
| Compound I+DLM+LZD | | | 5.03±0.18 | 2.94±0.22 | 2.65±0.31 |
| BDQ+DLM+LZD | | | 4.91±0.17 | 3.69±0.11 | 2.83±0.27 |
| BDQ+PMD+LZD | | | 3.29±0.18 | 0.55±0.15 | 0.48±0.00 |
| Compound I+PMD+LZD | | | 2.92±0.35 | 2.00±0.00 | Negative |
| D-16: 14 days post-infection; D0: date of administration and treatment; 4 weeks/8 weeks/12 weeks: 4 weeks/8 weeks/12 weeks post-administration. | | | | | |

Table 10: CFU counts in spleens of BALB/c mice at various time points

| Group | Average log10 CFU count | | | | |
|---|---|---|---|---|---|
| | D-16 | D0 | 4 weeks | 8 weeks | 12 weeks |
| Untreated group | 1.23±0.17 | 3.59±0.33 | 4.39±0.06 | 4.43±0.12 | 4.09±0.09 |
| HRZ | | | 0.70±0.00 | Negative | 2.04±0.00 |
| DLM | | | 2.97±0.14 | 2.23±0.21 | 2.17:1:0.27 |
| Compound I | | | 2.60±0.00 | Negative | Negative |
| Compound I+DLM | | | 2.85±0.15 | 2.00±0.00 | 0.30±0.00 |
| Compound I+DLM+LZD | | | 1.93±0.11 | 0.70±0.00 | 0.32±0.45 |
| BDQ+DLM+LZD | | | 1.72±0.23 | 1.43±0.28 | 0.24±0.24 |
| BDQ+PMD+LZD | | | 1.12±0.08 | Negative | 0.60±0.00 |
| Compound I+PMD+LZD | | | 1.70±0.00 | Negative | Negative |
| D-16: 14 days post-infection; D0: date of administration and treatment; 4 weeks/8 weeks/12 weeks: 4 weeks/8 weeks/12 weeks post-administration. | | | | | |

According to the above test results, the positive control group (HRZ group) achieved pulmonary sterilization in mice after 8 weeks of treatment; however, after 12 weeks of treatment, the lung colony count increased to 1.51 ($log_{10}$ CFU). The compound I+PMD+LZD group showed negative lung culture results after 12 weeks of treatment, and after 12 weeks of treatment, the activity of the compound I+PMD+LZD group was superior to that of the BDQ+PMD+LZD group.

In summary, various drug combinations comprising compound I exhibited excellent antibacterial activity in animal experiments. In particular, when compound I and PMD were present simultaneously, both the combination of compound I with PMD and the combination of compound I with PMD and LZD demonstrated stronger efficacy than the single-drug groups.

Although specific embodiments of the present disclosure have been described above, those skilled in the art should understand that these are merely illustrative, and various changes or modifications can be made to these embodiments without departing from the principle and essence of the present disclosure.

## Claims

1. A pharmaceutical combination, comprising: i) substance X, wherein the substance X is compound I or a pharmaceutically acceptable salt thereof; and ii) substance Y, wherein the substance Y is a second therapeutic agent or a pharmaceutically acceptable salt thereof, and the second therapeutic agent is pyrazinamide, linezolid, moxifloxacin, pretomanid, or delamanid; the structure of compound I is shown below:

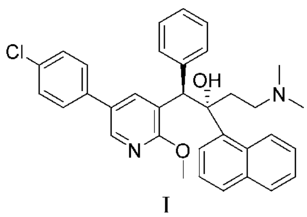

I

.

2. The pharmaceutical combination according to claim 1, wherein the substance X is a pharmaceutically acceptable salt of compound I; preferably, the substance X is a hydrochloride, sulfate, citrate, maleate, or fumarate salt of compound I; more preferably, the substance X is a fumarate salt of compound I;

   and/or, the second therapeutic agent is selected from pyrazinamide, moxifloxacin, delamanid, and pretomanid; preferably, the second therapeutic agent is delamanid or pyrazinamide;
   and/or, the pharmaceutical combination consists of the substance X and the substance Y;
   and/or, the substance X and the substance Y are administered simultaneously or separately.

**3.** The pharmaceutical combination according to claim 1 or 2, wherein the substance X is administered orally; and/or, the substance Y is administered orally.

**4.** A pharmaceutical composition A, comprising substance X, substance Y, and a pharmaceutically acceptable carrier or medium, wherein the substance X is compound I or a pharmaceutically acceptable salt thereof; the substance X is preferably a pharmaceutically acceptable salt of compound I; the substance X is more preferably a hydrochloride, sulfate, citrate, maleate, or fumarate salt of compound I; the substance X is further preferably a fumarate salt of compound I;

the substance Y is a second therapeutic agent or a pharmaceutically acceptable salt thereof, and the second therapeutic agent is pyrazinamide, linezolid, moxifloxacin, pretomanid, or delamanid; the second therapeutic agent is preferably pyrazinamide or delamanid;
the structure of compound I is shown below:

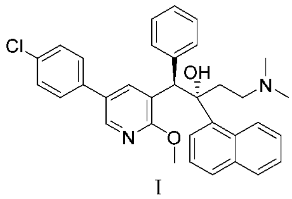

I .

**5.** The pharmaceutical composition A according to claim 4, wherein the pharmaceutical composition A is presented in an oral dosage form;
and/or, the pharmaceutical composition A consists of the substance X, the substance Y, and a pharmaceutically acceptable carrier or medium.

**6.** A pharmaceutical composition B, comprising a first pharmaceutical composition and a second pharmaceutical composition;

the first pharmaceutical composition comprises substance X and a pharmaceutically acceptable carrier or medium, wherein the substance X is compound I or a pharmaceutically acceptable salt thereof; the substance X is preferably a pharmaceutically acceptable salt of compound I; the substance X is more preferably a hydrochloride, sulfate, citrate, maleate, or fumarate salt of compound I; the substance X is further preferably a fumarate salt of compound I;
the second pharmaceutical composition comprises substance Y and a pharmaceutically acceptable carrier or medium, wherein the substance Y is a second therapeutic agent or a pharmaceutically acceptable salt thereof, and the second therapeutic agent is pyrazinamide, linezolid, moxifloxacin, pretomanid, or delamanid; the second therapeutic agent is preferably pyrazinamide or delamanid;
the structure of compound I is shown below:

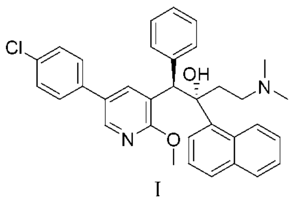

I .

**7.** The pharmaceutical composition B according to claim 6, wherein the first pharmaceutical composition is presented in an oral dosage form; and/or, the second pharmaceutical composition is presented in an oral dosage form.

**8.** The pharmaceutical composition B according to claim 7, wherein the pharmaceutical composition B consists of the first pharmaceutical composition and the second pharmaceutical composition.

**9.** A pharmaceutical combination, comprising

i) substance X, wherein the substance X is compound I or a pharmaceutically acceptable salt thereof;

ii) substance Y, wherein the substance Y is a second therapeutic agent or a pharmaceutically acceptable salt thereof, and the second therapeutic agent is pretomanid or delamanid; and
iii) substance Z, wherein the substance Z is linezolid or a pharmaceutically acceptable salt thereof;

the structure of compound I is shown below:

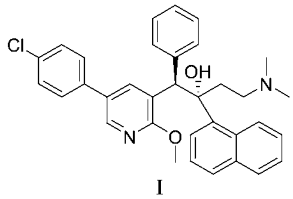

I

.

**10.** The pharmaceutical combination according to claim 9, wherein the substance X is a pharmaceutically acceptable salt of compound I; preferably, the substance X is a hydrochloride, sulfate, citrate, maleate, or fumarate salt of compound I; more preferably, the substance X is a fumarate salt of compound I;

and/or, the second therapeutic agent is pretomanid;
and/or, the pharmaceutical combination consists of the substance X, the substance Y, and the substance Z;
and/or, the substance X, the substance Y, and the substance Z are administered simultaneously or separately.

**11.** The pharmaceutical combination according to claim 9 or 10, wherein the substance X is administered orally; and/or, the substance Y is administered orally; and/or, the substance Z is administered orally.

**12.** A pharmaceutical composition C, comprising substance X, substance Y, substance Z, and a pharmaceutically acceptable carrier or medium, wherein the substance X is compound I or a pharmaceutically acceptable salt thereof; the substance X is preferably a pharmaceutically acceptable salt of compound I; the substance X is more preferably a hydrochloride, sulfate, citrate, maleate, or fumarate salt of compound I; the substance X is further preferably a fumarate salt of compound I;

the substance Y is a second therapeutic agent or a pharmaceutically acceptable salt thereof, and the second therapeutic agent is pretomanid or delamanid; the second therapeutic agent is preferably pretomanid;
the substance Z is linezolid or a pharmaceutically acceptable salt thereof;
the structure of compound I is shown below:

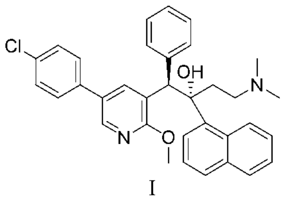

I

.

**13.** The pharmaceutical composition C according to claim 12, wherein the pharmaceutical composition C is presented in an oral dosage form; and/or, the pharmaceutical composition C consists of the substance X, the substance Y, the substance Z, and a pharmaceutically acceptable carrier or medium.

**14.** A pharmaceutical composition D, comprising a first pharmaceutical composition, a second pharmaceutical composition, and a third pharmaceutical composition;

the first pharmaceutical composition comprises substance X and a pharmaceutically acceptable carrier or medium, wherein the substance X is compound I or a pharmaceutically acceptable salt thereof; the substance X is preferably a pharmaceutically acceptable salt of compound I; the substance X is more preferably a hydrochloride, sulfate, citrate, maleate, or fumarate salt of compound I; the substance X is further preferably a fumarate salt of compound I;
the second pharmaceutical composition comprises substance Y and a pharmaceutically acceptable carrier or medium, wherein the substance Y is a second therapeutic agent or a pharmaceutically acceptable salt thereof,

and the second therapeutic agent is pretomanid or delamanid; the second therapeutic agent is preferably pretomanid;

the third pharmaceutical composition comprises substance Z and a pharmaceutically acceptable carrier or medium, wherein the substance Z is linezolid or a pharmaceutically acceptable salt thereof;

the structure of compound I is shown below:

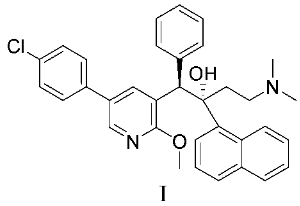

I

.

**15.** The pharmaceutical composition D according to claim 14, wherein the first pharmaceutical composition is presented in an oral dosage form;

and/or, the second pharmaceutical composition is presented in an oral dosage form;

and/or, the third pharmaceutical composition is presented in an oral dosage form.

**16.** The pharmaceutical composition D according to claim 15, wherein the pharmaceutical composition D consists of the first pharmaceutical composition, the second pharmaceutical composition, and the third pharmaceutical composition.

**17.** Use of the pharmaceutical combination according to any one of claims 1 to 3 and 9 to 11, the pharmaceutical composition A according to claim 4 or 5, the pharmaceutical composition B according to any one of claims 6 to 8, the pharmaceutical composition C according to claim 12 or 13, or the pharmaceutical composition D according to any one of claims 14 to 16 in the manufacture of a medicament for the prevention or treatment of a disease caused by *Mycobacterium tuberculosis.*

**18.** Use of substance X in the manufacture of a medicament for the prevention or treatment of a disease caused by *Mycobacterium tuberculosis,* the substance X being compound I or a pharmaceutically acceptable salt thereof, wherein the substance X is used in combination with substance Y, wherein the substance Y is a second therapeutic agent or a pharmaceutically acceptable salt thereof, and the second therapeutic agent is pyrazinamide, linezolid, moxifloxacin, pretomanid, or delamanid; the second therapeutic agent is preferably pyrazinamide or delamanid; the structure of compound I is shown below:

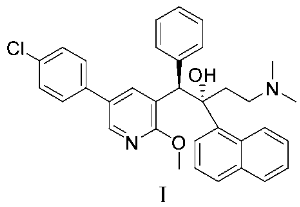

I

.

**19.** Use of substance X in the manufacture of a medicament for the prevention or treatment of a disease caused by *Mycobacterium tuberculosis,* the substance X being compound I or a pharmaceutically acceptable salt thereof, wherein the substance X is used in combination with substance Y and substance Z, wherein the substance Y is a second therapeutic agent or a pharmaceutically acceptable salt thereof, and the second therapeutic agent is pretomanid or delamanid; the substance Z is linezolid or a pharmaceutically acceptable salt thereof; the structure of compound I is shown below:

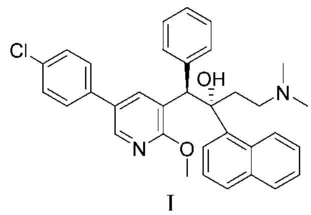

I

.

## INTERNATIONAL SEARCH REPORT

International application No.
**PCT/CN2024/119801**

**A. CLASSIFICATION OF SUBJECT MATTER**

A61K 45/06(2006.01)i; A61K 31/4418(2006.01)i; A61K 31/4545(2006.01)i; A61P 31/06(2006.01)i

According to International Patent Classification (IPC) or to both national classification and IPC

**B. FIELDS SEARCHED**

Minimum documentation searched (classification system followed by classification symbols)

IPC:A61K 45/-; A61K 31/-; A61P 31/-

Documentation searched other than minimum documentation to the extent that such documents are included in the fields searched

Electronic data base consulted during the international search (name of data base and, where practicable, search terms used)

CNTXT; VEN; ENTXT; WOTXT; USTXT; EPTXT; GBTXT; JPTXT; CNMED; MEDNPL; CNKI: 万方, WANFANG; 百度学术, BAIDU SCHOLAR; 读秀, DUXIU; STN; Web of Science: 舒达吡啶; 吡嗪酰胺; 利奈唑胺; 莫西沙星; 普瑞玛尼; 德拉马尼; 结核病; 结核分枝杆菌; 化合物I结构检索; 广州嘉越医药科技有限公司; 首都医科大学附属北京胸科医院; 上海嘉坦医药科技有限公司; 陆宇; 付雷; 姚蓉; 张炜焱; 王彬; 陈曦; 王宁; 丁杨明; 张蕾; 李宗瑞; 陈小宁; 李磊; 李永国; tuberculosis; MTB; WX-081; sudapyridine; Pyrazinamide; Moxifloxacin; Linezolid; Pretomanid; Delamanid; 98-96-4; 151096-09-2; 165800-03-3; 187235-37-6; 681492-22-8; GUANGZHOU JOYO PHARMATECH; BEIJING CHEST HOSPITAL AFFILIATED OFCAPITAL MEDICAL UNIVERSITY; SHANGHAI JIA TAN PHARMATECH.

**C. DOCUMENTS CONSIDERED TO BE RELEVANT**

| Category* | Citation of document, with indication, where appropriate, of the relevant passages | Relevant to claim No. |
|---|---|---|
| X | CN 116763807 A (BEIJING CHEST HOSPITAL, CAPITAL MEDICAL UNIVERSITY) 19 September 2023 (2023-09-19)<br>description, paragraphs 6-7, 12-13, 16, and 17 | 1-8, 17-19 |
| PX | CN 117243956 A (GUANGZHOU INSTITUTE OF BIOMEDICINE AND HEALTH, CHINESE ACADEMY OF SCIENCES et al.) 19 December 2023 (2023-12-19)<br>description, paragraphs 19 and 23, and embodiments 3-4 | 1-3, 17 |
| A | CN 105330595 A (MEDSHINE DISCOVERY INC. et al.) 17 February 2016 (2016-02-17)<br>claims 1-14 | 1-19 |
| A | CN 108473428 A (SHANGHAI JIATAN PHARMATECH CO., LTD. et al.) 31 August 2018 (2018-08-31)<br>abstract | 1-19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 December 2024** | **27 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/119801**

**C. DOCUMENTS CONSIDERED TO BE RELEVANT**

| Category* | Citation of document, with indication, where appropriate, of the relevant passages | Relevant to claim No. |
|---|---|---|
| A | WO 2011128628 A2 (PROBIO ASA et al.) 20 October 2011 (2011-10-20)<br>claims 1-10 | 1-19 |
| A | HUANG, Zhigang et al. "Discovery and Preclinical Profile of Sudapyridine (WX-081), a Novel Anti-Tuberculosis Agent"<br>*Bioorganic & Medicinal Chemistry Letters,* Vol. 71, 01 September 2022 (2022-09-01), 1-7 ISSN: 0960-894X,<br>abstract | 1-19 |

Form PCT/ISA/210 (second sheet) (July 2022)

**INTERNATIONAL SEARCH REPORT**
**Information on patent family members**

International application No.
**PCT/CN2024/119801**

| Patent document cited in search report | | | Publication date (day/month/year) | Patent family member(s) | | | Publication date (day/month/year) |
|---|---|---|---|---|---|---|---|
| CN | 116763807 | A | 19 September 2023 | None | | | |
| CN | 117243956 | A | 19 December 2023 | None | | | |
| CN | 105330595 | A | 17 February 2016 | None | | | |
| CN | 108473428 | A | 31 August 2018 | WO | 2017121323 | A1 | 20 July 2017 |
| | | | | TW | 201725198 | A | 16 July 2017 |
| | | | | TWI | 714702 | B | 01 January 2021 |
| WO | 2011128628 | A2 | 20 October 2011 | GB | 201006175 | D0 | 02 June 2010 |
| | | | | WO | 2011128628 | A3 | 08 November 2012 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023112288517
- WO 2017121323 A1


### Non-patent literature cited in the description


- Pharmacopoeia of the People's Republic of China. 2020, vol. IV
- **RAYMOND C ROWE**. Handbook of Pharmaceutical Excipients. 2009
- **BERGE et al.** Pharmaceutical Salts. *Journal of Pharmaceutical Science*, 1977, vol. 66, 1-19
- Handbook of Pharmaceutical Salts: Properties, Selection, and Use. Wiley-VCH, 2002
- *CHEMICAL ABSTRACTS*, 98-96-4
- *CHEMICAL ABSTRACTS*, 151096-09-2
- *CHEMICAL ABSTRACTS*, 165800-03-3
- *CHEMICAL ABSTRACTS*, 187235-37-6
- *CHEMICAL ABSTRACTS*, 681492-22-8